(19) Europäisches Patentamt
European Patent Office
Office européen des brevets

(11) EP 2 103 936 A1

(12) EUROPEAN PATENT APPLICATION

(43) Date of publication:
**23.09.2009 Bulletin 2009/39**

(51) Int Cl.:
***G01N 33/542*** *(2006.01)* ***G01N 33/533*** *(2006.01)*

(21) Application number: **08290262.8**

(22) Date of filing: **19.03.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(71) Applicant: **Institut Pasteur**
**75015 Paris (FR)**

(72) Inventors:
- **Bedouelle Hugues**
  **75015 Paris (FR)**
- **Brient-Litzler Elodie**
  **75015 Paris (FR)**

(74) Representative: **Thomas, Dean et al**
**Cabinet Ores**
**36 rue de St Pétersbourg**
**75008 Paris (FR)**

(54) **Reagentless fluorescent biosensors comprising a designed ankyrin repeat module and methods of their design and use**

(57) The invention relates to a reagentless peptide biosensor capable of recognising at least one ligand. This biosensor comprises at least one ankyrin repeat module and with this ankyrin repeat at least one cysteine residue coupled to a fluorophore. In a preferred embodiment the fluorophore is linked to a residue which has an altered solvent accessible surface area when the biosensor binds to its ligand. The invention also relates to various methods of designing such reagentless biosensors and to multivalent biosensors capable of binding with increased avidity to a ligand or capable of recognising multiple ligands simultaneously.



Printed by Jouve, 75001 PARIS (FR)

## Description

The present invention relates to improved reagentless fluorescent (RF) biosensors; in particular the present invention relates to reagentless fluorescent biosensors comprising at least one ankyrin repeat and a fluorophore.

A molecular biosensor transforms a specific molecular signal into an electrical signal and comprises several modules: a recognition module, which is also called a receptor and can be biological or biomimetic; a transduction module, which transforms the recognition event into a measurable signal; and a module of data evaluation. The recognition and transduction modules are integrated into a compact device with molecular dimensions (Lowe 1984). A biosensor can function without additional reagent, can provide quantitative analytical information and follow the concentration of its target, called the analyte continuously. Important characteristics of a biosensor are its specificity and selectivity, the sensitivity, linearity and speed of the response, the dynamic range of the measurements, the possibility of calibration and accuracy (Thevenot et al. 2001).

The physical nature of the measurable signal can be very diverse (Morgan et al. 1996). Fluorescence is an optical signal which allows one to detect molecular interactions with great sensitivity. The transduction is based on a variation of the fluorescence properties of the biosensor when it interacts with its analyte (Altschuh et al. 2006). The fluorescence of a protein biosensor can be intrinsic, e. g. provided by its component residues of tyrosine and tryptophan, or extrinsic, e. g. provided by the chemical coupling of fluorescent groups. The coupling of several fluorophores to a unique molecule of biosensor can be beneficial but is usually difficult to implement (Smith et al. 2005). Although intrinsic protein fluorescence can be used to study molecular interactions in purified experimental systems, extrinsic fluorescence is preferable to monitor specific interactions in complex media, without interference from other protein components (Foote and Winter 1992).

The changes of fluorescence that occur upon recognition between a RF biosensor and its analyte, result from different interactions between the fluorescent group and its environment in the free and bound forms of the biosensor. The binding of the analyte can occur in the neighborhood of the fluorescent group and directly modify its environment. Alternatively, the binding of the analyte can induce a conformational change of the biosensor and thus the interaction between the fluorescent group and the receptor indirectly. The inventors and others have used the first mechanism to create RF biosensors from antibodies, first when the three-dimensional structure of the complex with their antigen is known and then in the absence of such a knowledge (Renard et al. 2002; Renard et al. 2003; Jespers et al. 2004; Renard and Bedouelle 2004). This work also formed the basis of WO2001/065258 which described such antibody based biosensor molecules.

Other groups used the second mechanism to create biosensors from periplasmic binding proteins (de Lorimier et al. 2002). In both cases, a cysteine residue is introduced by site-directed mutagenesis in a pre-determined position of the receptor and a fluorophore is chemically coupled to this unique residue of cysteine.

Antibodies are perfectly suited to provide the recognition module of biosensors since they can be directed against any antigen. However, they have several intrinsic limitations. The single-chain variable fragments (scFv), which serve as the starting molecules for the construction of biosensors, often have insufficient conformational stability and limited half-lives to be suitable for prolonged use or use in harsh conditions. They contain two disulfide bonds, one in each variable domain. Therefore, when produced in a prokaryote they must be exported into the oxidizing medium of the bacterial periplasm to allow permissive conditions for the formation of their disulfide bonds and their folding in a functional form. The necessity of periplasmic expression limits the yield of total peptide production. In addition the mutant cysteine in the scFv to which the fluorophore is chemically coupled, often needs to be reactivated by a mild reduction before coupling. This reduction partially attacks the disulfide bonds of the fragment and further decreases the production yield of a fluorescent scFv conjugate.

These same problems of expression and stability are encountered with most recombinant antibodies and have slowed down their exploitation in various domains of application. These problems have led several groups to develop alternative families of antigen binding proteins, by engineering proteins that have (i) a stable polypeptide scaffold, (ii) are devoid of cysteine residues and disulfide bonds, and (iii) are well expressed in *Escherichia coli.* These new families of antigen binding proteins can replace antibodies in many of their applications (Mathonet and Fastrez 2004; Binz et al. 2005).

The family of the Designed Ankyrin Repeat Proteins (Darpins) illustrate the potential of these artificial families of antigen binding proteins. The repeated ankyrin modules are present in thousands of proteins from all phyla and involved in recognitions between proteins (Mosavi et al. 2004; Li et al. 2006). Consensus sequences of these modules have been established and the corresponding consensus proteins shown to possess remarkable biophysical properties. Their stability ($T_{1/2}$ > 65 °C), solubility (several mM) and production yield in the cytoplasm of E. *coli* (up to 200 mg/L of culture in flask) are more favorable than those of the antibody fragments (Mosavi et al. 2002; Binz et al. 2003; Kohl et al. 2003). Combinatorial libraries of Darpins have been generated by randomization of residues that potentially belong to the paratope (antigen binding site), and assemblage of a random number of ankyrin modules between defined N- and C-terminal modules (Binz et al. 2003). These libraries were used to select Darpins that bound specific protein antigens, by ribosome display (Zahnd et al. 2007). Variants with high affinities and specificities have been obtained against the maltose binding protein (MalE) from *E. coli,* eukaryotic and prokaryotic kinases, the cancer marker Her2, the TEV protease and the CitS membrane proteins (Binz et al. 2004; Amstutz et al. 2005; Zahnd et al. 2006; Huber et al. 2007; Zahnd et al. 2007, Kawe et al, 2006). These studies have shown that the Darpins can be used as inhibitors of biological functions *in vivo*, for the targetting of tumors *in vivo*, and for diagnostic applications *in vitro.*

In particular the Inventors have now developed a new type of reagentless biosensors which incorporate the advantages of Darpins; the inventors have also developed methods to design and produce such new reagentless biosensors. These overcome the problems associated with antibody based reagentless biosensors, such as poor physicochemical properties and complex production regimes. In addition the inventors have unexpectedly found that the rate of successful creation of Darpin biosensors is greater than with their previous work using antibodies and fragments thereof. Also the inventors have found that Darpin based RF biosensors according to the current invention have higher sensitivity in comparison to the RF biosensors based upon antibodies and antibody fragments that they previously developed.

The inventors have therefore developed a novel method to generate RF biosensors and describe herein such novel RF biosensors and rules for the design of RF biosensors from Darpins when the three-dimensional structure of the complex with the ligand is known or unknown.

Therefore the present invention relates to a reagentless peptide biosensor for at least one ligand, comprising:

at least one ankyrin repeat module;
at least one cysteine residue coupled to a fluorophore.

In the current Application an ankyrin repeat module is one which consists of one or more ankyrin repeat.

The ankyrin repeat, a 33-residue sequence motif, was first identified in the yeast cell cycle regulator Swi6/Cdc10 and the *Drosophila* signalling protein Notch (Breeden and Nasmyth 1987), and was eventually named after the cytoskeletal protein ankyrin, which contains 24 copies of this repeat (Lux et al. 1990). Subsequently, ankyrin repeats have been found in many proteins spanning a wide range of functions.

The individual ankyrin repeats in the ankyrin repeat module can be identical or different. Each of these ankyrin repeats may each comprise a fluorophore or not and in each ankyrin repeat the flurophore may be attached to the same or a different residue.

In particular the cysteine residue is present at a position of the biosensor whose solvent accessible surface area is altered when said biosensor binds to said at least one ligand but which does not directly interact therewith.

The inventors therefore provide a new class of reagentless biosensor which has the advantages of a reagentless biosensor, namely a biosensor which can function without additional reagent and can provide quantitative analytical information and follow the concentration of its analyte continuously together with the more robust bio/physico-chemical properties of Darpins. They have validated this new class of RF biosensors with the known Darpin DarpOff7, a Darpin which is directed against the MalE protein (Binz et al. 2004).

The inventors have shown that several variants of such Darpin based biosensors work using the MalE protein from *Escherichia coli* as a model target.

Such reagentless fluorescent biosensors can be used in different formats: in solution, in the form of protein chips, or at the tip of optical micro- or nano-fibers. They could be used for the continuous quantification of antigens in complex mixtures, without any prior labelling of the proteins under analysis. In health, they could be used for the bed side monitoring of patients, the controlled continuous delivery of drugs, the control of artificial organs, some diagnostics, in situ measurements during surgical operations, and the detection of doping drugs. In industry, they could be used for the monitoring of reactions and processes, food control, and pharmacokinetic studies. In environment and civil or military defence, they could be used for the monitoring of pathogenic, toxic or polluting agents. In fundamental research, they could be used in proteomics, for the profiling of cells, tissues or body fluids; in the biology of single cells, to continuously measure the concentration of an antigen within a single living cell; in neuro-chemistry and neuro-sciences, to measure the intra-cerebral concentration of neuro-peptides in response to external stimuli.

In particular the reagentless biosensor may be derived from a parental binding protein for said ligand.

In the current Application a parental binding protein refers to any protein known or suspected to have binding affinity for a given ligand and from which the binding portion of this protein can be isolated and used in the construction of a reagentless biosensor according to the current invention.

In particular each ankyrin repeat is a 30 to 35 residue polypeptide comprising a canonical helix-loop-helix-beta hairpin/loop fold structure.

In particular this biosensor comprises at least one ankyrin repeat which consists of SEQ ID NO: 3 or SEQ ID NO: 8 or a sequence of at least 60% similarity therewith.

These percentages of sequence similarity defined herein were obtained using the BLAST program (blast2seq, default parameters) (Tatutsova and Madden, FEMS Microbiol Lett., 1999, 174, 247-250).

SEQ ID NO: 3 or SEQ ID NO:8 represent consensus sequences of the ankyrin repeat.

Such percentage sequence similarity is derived from a full length comparison with SEQ ID NO:3 or SEQ ID NO:8, as detailed herein; preferably these percentages are derived by calculating them on an overlap representing a percentage of length of SEQ ID NO:3 or SEQ ID NO:8.

In particular the biosensor comprises at least one ankyrin repeat which has at least 80% similarity with SEQ ID NO:3 or SEQ ID NO: 8.

In particular the biosensor comprises at least one ankyrin repeat which has at least 95% similarity with SEQ ID NO:3 or SEQ ID NO: 8.

In particular the biosensor according to the current invention has a fluorophore coupled to an ankyrin repeat of the ankyrin repeat module at a position selected from:

(i) residues 2, 3, 5, 13, 14, 26 and 33; or
(ii) residues 1, 4, 6, 12, 15, 25, 27, 32,

the residues being changed to cysteine residues if they are not already cysteine residues.

In particular the biosensor according to the present invention has a fluorophore coupled to one residue of SEQ ID NO: 3 or SEQ ID NO: 8, selected from the sets (i) and (ii) of the residues above.

In particular the biosensor or its parental binding protein may comprise at least an N-terminal capping ankyrin repeat and/or a C-terminal capping ankyrin repeat.

In particular the N-terminal capping ankyrin repeat consists of SEQ ID NO: 4 and the C-terminal capping ankyrin repeat consists of SEQ ID NO: 5.

In particular in the biosensor according to the present invention the at least one cysteine residue is either present in said biosensor or is substituted with another suitable residue. Wherein the at least one cysteine residue or the substituted residue has a solvent accessible surface area which is altered when the biosensor binds to the ligand, but which does not directly interact directly therewith.

In particular the residue forms an indirect contact with the ligand via at least one water molecule.

Alternatively the residue does not contact the ligand, neither directly nor indirectly.

In particular the fluorophore is selected from the group consisting of: 6-acryloyl-2-dimethylaminophtalene (acrylodan), 4-chloro-7-nitrobenz-2-oxa-1,3-diazole (CNBD),5-iodoacetamidoflurescein (5-IAF), (N-((2-(iodoacetoxy) ethyl)-N-methyl)amino-7-nitrobenz-2-oxa-1,3-diazole (IANBD ester), Cy3, Cy5 or a fluorophore having an aliphatic chain of 1 to 6 carbon atoms.

A fluorophore, is a component of a molecule which causes a molecule to be fluorescent. It is a functional group in a molecule which will absorb energy of a specific wavelength and re-emit energy at a different (but equally specific) wavelength. The amount and wavelength of the emitted energy depend on both the fluorophore and the chemical environment of the fluorophore.

In particular the biosensor is in soluble form.

In particular the biosensor is immobilized on a suitable solid support.

The present invention also relates to a biosensor which consists of SEQ ID NO: 2 in which one of residues 23, 45, 46, 53, 111, 112, 114, 122, 123 and 125 has been substituted with a cysteine residue and coupled to a fluorophore. The relationship of these residues to the ankyrin repeat consensus sequence is shown in figure 8.

The present invention also relates to a protein-based chip, characterized in that it consists of a solid support on which at least one biosensor as described in the current Patent Application is immobilized.

The present invention also relates to a solution comprising at least one biosensor as described in the current Patent Application.

The present invention also relates to an optical fibre comprising at a first end thereof at least one biosensor as described in the current Patent Application and comprising at a second end thereof means to attach the optical fibre to a device configured to receive and interpret the output of the at least one biosensor.

The present invention also relates to a method for producing biosensors as described in the current Patent Application, **characterized in that** it comprises the following steps:

(a) selecting at least one residue of the biosensor by searching for the residues which have a solvent accessible surface area (ASA) which is modified by the binding of said at least one ligand, when use is made of spheres of increasing radius of 1.4 to 30Å, for the molecule of said solvent; and which (i) are in contact with said ligand via a water molecule, or (ii) do not contact said ligand;
(b) mutating by site-directed mutagenesis at least one of the residues selected in (a) to a Cys residue when said residue is not naturally a Cys residue, and
(c) coupling the Sγ atom of at least one Cys residue obtained in (a) or in (b) to a fluorophore.

In particular the preparation method is **characterised in that** the biosensor comprises at least a portion of a parental protein known to bind the ligand.

In particular the preparation method is **characterized in that**, prior to step (a), it comprises a step of modelling the biosensor or its parental protein and/or the ligand and/or the biosensor/parental protein-ligand complexes.

In particular this modelling may be either by means of *ab initio* protein structure modelling programmes such as MODELLER or swissmodeller; or comparative protein modelling using previously solved structures as starting points. Alternatively using 3D models derived from protein crystallography, NMR or other means.

The present invention also relates to a method for producing biosensors as described in the current Patent Application, which comprises the following steps:

(a1) identifying the active site of the biosensor by mutagenesis of the set, or of a subset, of the residues of the biosensor, and determining the variations in the parameters of interaction with the ligand ($K_d$ (which represents the equilibrium constant), $k_{on}$ (which represents the association rate constant) and $k_{off}$ (which represents the dissociation rate constants,) which are due to each mutation or to limited groups of mutations;
(b1) selecting the Cys residues, or the residues to be mutated into cysteine, from the residues of the biosensor which are located in the proximity of the residues of the active site along the sequence;
(c1) mutating by site-directed mutagenesis at least one of the residues selected in (b1) to a Cys residue when said residue is not naturally a Cys residue; and
(d1) coupling the S$\gamma$ atom of at least one Cys residue obtained in (b1) or in (c1) to a fluorophore.

In particular the preparation method is **characterised in that** the biosensor comprises at least a portion of a parental protein known to bind the ligand.

In particular in step (b1) the selected residues are adjacent to the residues of the binding site along the peptide backbone (residues -1 and +1).

The present invention also relates to a method for preparing biosensors as described in the current Patent Application which comprises at least two ankyrin repeat modules, **characterized in that** it comprises the following steps:

(a2) identifying the binding site of a first ankyrin repeat module by scanning mutagenesis of the set or of a subset of the residues of said first ankyrin repeat module, and determining the variations in the parameters of interaction with the ligand ($K_D$, $k_{on}$, $k_{off}$) which are due to each mutation or to limited groups of mutations;
(b2) selecting the Cys residues, or the residues to be mutated to Cys, from the residues of a second ankyrin repeat module which are in positions equivalent to the residues of the binding site or are located in the proximity of the residues of the binding site of said first ankyrin repeat module;
(c2) mutating by site-directed mutagenesis at least one of the residues selected in (b2) to a Cys residue when said residue is not naturally a Cys residue; and
(d2) coupling the S$\gamma$ atom of at least one Cys residue obtained in (b2) or in (c2) to a fluorophore.

This method allows the modification of residues found to be important in one ankyrin repeat module to be modified in a second module, so broadening the residues which can be altered so as to derive a reagentless biosensor of the required characteristics according to the current invention.

In particular the residues in the binding site of the first ankyrin domain are identified by means of *ab initio* protein structure modelling programmes such as MODELLER or swissmodeller; or comparative protein modelling using previously solved structures as starting points. Alternatively using 3D models derived from protein crystallography, NMR or other means.

In particular the preparation method is **characterized in that**, prior to step (a), (a1) or (a2), the nonessential Cys residues of the biosensor are substituted with Ser or Ala residues by site-directed mutagenesis.

In particular the preparation method is **characterized in that**, in step (c), (d1) or (d2), said fluorophore is selected from the group consisting of: IANBD, CNBD, acrylodan, 5-iodoacetamidofluorescein or a fluorophore having an aliphatic chain of 1 to $\gamma$ carbon atoms.

In particular the preparation method is **characterized in that**, prior to step (c), (d1) or (d2), the mutated biosensor obtained in step (b), (c1) or (c2) is subjected to a controlled reduction.

In particular the preparation method is **characterized in that**, after step (c), (d1) or (d2), it comprises an additional step (d), (e1) or (e2) for purifying the biosensor.

In particular the preparation method is **characterized in that**, after step (d), (e1) or (e2), it comprises an additional step for (i) measuring the equilibrium constant ($K_D$ or $K'_D$) for said purified biosensor, or the dissociation ($K_{off}$) and association ($k_{on}$) rate constants for the receptor and ligand; and (ii) measuring the fluorescence variation of said biosensor or ligand binding.

In particular the preparation method is **characterized in that**, after step (c), (d1) or (d2) or step (d), (e1) or (e2), it comprises an additional step for immobilizing the biosensor on a solid support.

The present invention also relates to the use *in vitro,* of a biosensor as described in the present Patent Application, for applications comprising detecting, assaying and locating said at least one ligand.

In particular the use of the biosensors as described in the present Patent Application is for screening protein libraries.

In particular the use of the biosensors as described in the present Patent Application is for sorting molecules.

In particular the use of the biosensors as described in the present Patent Application is for sorting cells.

In particular the use of the biosensors as described in the present Patent Application is for producing protein-based chips.

The present invention also relates to a reagent for detecting, assaying or locating ligands, **characterized in that** it includes at least one biosensor as described in the current Patent Application.

The present invention also relates to a method for detecting, assaying or locating a ligand in a heterogeneous sample, **characterized in that** it comprises bringing the heterogeneous sample into contact with at least one reagent as described in the current Patent Application.

The present invention also relates to a kit for detecting, assaying or locating ligands, **characterized in that** it includes at least one reagent as described in the current Patent Application.

The present invention also relates to a kit for screening for inhibitors of the ligand/receptor interaction, **characterized in that** it includes at least one reagent as described in the current Patent Application.

The present invention also relates to a reagentless peptide biosensor for at least one ligand, wherein said biosensor comprises at least two ankyrin repeat modules and each of said ankyrin repeat modules comprises at least two cysteine residues, and wherein a fluorophore is attached to a first cysteine residue in each of said ankyrin repeat modules, and wherein each of said ankyrin repeat modules is linked to at least one other of said ankyrin repeat modules via a disulfide bond between a second cysteine residue in each of said ankyrin repeat modules.

The present invention therefore also relates to bivalent or bifunctional Darpin dimers comprising two or more biosensor modules linked by a disulfide bond. Such bifunctional Darpins enlarge the potential functionality of the reagentless biosensors according to the current invention. In particular two or more homologous modules can increase the avidity of the biosensor for the ligand or two or more heterologous modules allow a single biosensor molecule to visualise two or more ligands simultaneously. Preferably the cysteine residues involved in forming the disulfide bond between two or more modules should be outside of the ligand binding site so as to not interfere with the biosensor/ligand interaction.

In particular the at least two ankyrin repeat modules are homologous.

Alternatively the at least two ankyrin repeat modules are heterologous.

In particular each of said heterologous ankyrin repeat modules comprise a different fluorophore.

In particular each of said first cysteine residue is present at a position of each said ankyrin repeat module whose solvent accessible surface area is altered when said biosensor binds to said at least one ligand but which does not directly interact therewith.

For a better understanding of the invention and to show how the same may be carried into effect, there will now be shown by way of example only, specific embodiments, methods and processes according to the present invention with reference to the accompanying drawings in which:

**Figure 1.** Shows the positions of the coupling sites in the structure of DapOff7.
**Figure 2.** Shows the titration of DarpOff7 conjugates by MalE, monitored by fluorescence.
**Figure 3.** Shows the selectivity and specificity of the fluorescence signal for the DarpOff7(N45ANBD) conjugate.
**Figure 4.** Shows the quenching of the DarpOff7(N45ANBD) fluorescence by KI.
**Figure 5.** Shows the effects of the concentration in serum on the fluorescence signals for the DarpOff7(N45ANBD) conjugate.
**Figure 6.** Shows the ranking of the DarpOff7 conjugates according to their relative sensitivities $s_r$ at 25 °C in buffer L1.
**Figure 7.** Shows the ranking of the DarpOff7 conjugates according to their lower limit of detection at 25 °C in buffer L1.
**Figure 8.** Shows the relative positions of the coupling sites in the ankyrin repeats.

There will now be described by way of example a specific mode contemplated by the Inventors. In the following description numerous specific details are set forth in order to provide a thorough understanding. It will be apparent however, to one skilled in the art, that the present invention may be practiced without limitation to these specific details. In other instances, well known methods and structures have not been described so as not to unnecessarily obscure the description.

## <u>EXAMPLE 1</u> - Materials and Methods

### 1.1 Analysis of the structural data

The crystal structure of the complex between DarpOff7 and MalE (PDB 1 SVX) was analyzed with the What If program (Vriend 1990).

The solvent accessible surface area (ASA) were calculated with the ACCESS routine and a radius of the solvent sphere equal to 1.4 Å (Ångström). The contact residues between DarpOff7 and MalE were identified with the ANACON routine, using extended Van der Waals radii as described (Rondard and Bedouelle 1998). Water molecules bridging DarpOff7 and MalE were identified with the subroutine NALWAT. The three-dimensional structures of the cysteine mutants of DarpOff7 were modeled with the mutation prediction program of the What If web interface (http://swift.cmbi.kun.nl/whatif/).

### 1.2 Materials

LB medium, the *E. coli* strains XL1-Blue (Bullock et al. 1987) and AVB99 (Smith et al. 1998); plasmids pQEMBP (SEQ ID NO: 6), pAT224 (SEQ ID NO: 7), pQEOFF7 (SEQ ID NO: 9) and the DarpOff7 (SEQ ID NO: 1) (Binz et al. 2004) have been described. pQEMBP (SEQ ID NO: 6) codes for the maltose binding protein MalE from *E. coli.* pAT224 (SEQ ID NO: 7) codes for a hybrid bt-MalE between a peptide that can be biotinylated *in vivo* by *E. coli,* and MalE. DarpOff7 (SEQ ID NO: 1) codes for a Darpin, DarpOff7, directed against MalE.

All the recombinant proteins carry a hexahistidine tag.

Buffer H was 500 mM NaCl, 50 mM Tris-HCl (pH 7.5); buffer Ml, 150 mM NaCl, 50 mM Tris-HCl (pH 7.5); buffer L1, 50 mM NaCl, 20 mM Tris-HCl (pH 7.5); buffer M2, 0.005 % (v/v) P20 surfactant (Biacore) in buffer M1; buffer L2, 0.005 % (v/v) P20 surfactant in buffer L1; buffer M3, 5 mM dithiothreitol (DTT) in buffer M2. Phosphate buffer saline (PBS), calf serum, hen egg white lysozyme, DTT, Tween 20 and tris(2-carboxyethyl)phosphine (TCEP) were purchased from Sigma, bovine serum albumin (BSA) from Roche, N-((2-(iodoacetoxy)ethyl)-N-methyl)amino-7-nitrobenz-2-oxa-1,3-diazole (IANBD ester) from Invitrogen. A stock solution of the IANBD ester was made at a concentration of 10 mg/mL in dimethylformamide. Ampicillin was used at a concentration of 100 μg/mL and chloramphenicol at 10 μg/mL.

### 1.3 Mutagenesis and gene synthesis

Changes of residues were constructed in the DarpOff7 protein (SEQ ID NO: 2) at the genetic level, by mutagenesis of pQEOff7 open reading frame (SEQ ID NO: 9) with the Quickchange II site directed mutagenesis kit (Stratagene). As Darpins consist of repeated modules of the ankyrin repeat polypeptide and encoded by similarly repeated segments of DNA, the mutagenic primers were designed so that their 3'-nucleotide or the preceding nucleotide was specific of the targeted segment and their elongation by DNA-polymerase could not occur on another repeated segment. Mutation K122C could not be obtained in this way. To obtain this residue change the inventors therefore used the degeneracy of the genetic code to design a mutant allele of the *off7* gene that was devoid of extensive repetitions. The mutant allele, *off71 (SEQ ID NO: 10)*, was synthesized by Genecust (Evry, France) and used to construct mutations K68C and K122C.

### 1.4 Protein production, purification and characterization

The MalE protein was produced in the cytoplasm of the recombinant strain XL1-Blue(pQEMBP), bt-MalE in strain AVB99(pAT224) and DarpOff7 and its mutant derivatives in XL1-Blue(pQEOff7) and its mutant derivatives, as described (Binz et al. 2003; Binz et al. 2004). They were purified through their hexahistidine tag by affinity chromatography on a column of fast-flow Ni-NTA resin, as recommended by the manufacturer (Qiagen).

The purification fractions were analyzed by SDS-PAGE, with the NuPAGE Novex system, MES buffer and See blue pre-stained standards (all from Invitrogen). Equal amounts of protein were loaded on the gels after heat denaturation either in the presence or in the absence of 2.5 % (v/v, 0.4 M) 2-mercaptoethanol. The gels were stained with Coomassie blue and the protein bands were quantified with the Un-scan-it software (Silk Scientific).

The fractions that were pure after SDS-PAGE in reducing conditions (> 98 % homogeneous), were pooled and kept at -80 °C. The protein concentrations were measured by absorbance spectrometry, with coefficients of molar extinction, $\varepsilon_{280}$(MalE) = 66350 $M^{-1}$ $cm^{-1}$, $\varepsilon_{280}$(bt-MalE) = 71850 $M^{-1}$ $cm^{-1}$, and $\varepsilon_{280}$(DarpOff7)= 16960 $M^{-1}$ $cm^{-1}$, calculated as described (Pace et al. 1995). Aliquots of the wild type DarpOff7(wt) and its mutant derivative DarpOff7(N45C) were analyzed by mass spectrometry after extensive dialysis against 65 mM ammonium bicarbonate and lyophilization, as described (Renard et al. 2002). All the binding experiments were performed at 25 °C.

### 1.5 Indirect ELISA

ELISA experiments were performed in buffer M1 and micro-titer plates as described (Harlow and Lane 1988), except that the wells of the plates were washed three times with 0.05% (v/v) Tween 20 in buffer M1 and three times with buffer M1 alone between each step. The wells were coated with 0.5 μg $ml^{-1}$ DarpOff7 and blocked with 3% BSA (w/v). The immobilized Darpin was incubated with bt-MalE and varying concentrations of potassium iodide KI in 1% BSA for 1 h at 25 °C. bt-MalE was omitted in the blank wells. The captured molecules of bt-MalE were revealed with a conjugate between streptavidin and alkaline phosphatase, and p-nitrophenyl phosphate as a substrate (all from Sigma-Aldrich). The absorbance at 405 nm, $A_{405}$, was measured and corrected by subtraction of the blank.

### 1.6 Fluorophore coupling

The cysteine mutants of DarpOff7 were reduced with 5 mM DTT for 30 min at 30 °C with gentle shaking and then transferred into PBS by size exclusion chromatography with a PD10 column (GE Healthcare). The thiol-reactive fluorophore IANBD ester was added in > 5:1 molar excess over the Darpin and the coupling reaction was carried out for 2 hours at 30 °C with gentle shaking. The denatured proteins were removed by centrifugation for 30 min at 10000 g, 4 °C. The conjugate was separated from the unreacted fluorophore by chromatography on a Ni-NTA column and elution with 100 mM imidazole in buffer H. The coupling yield $y_c$, i.e. the average number of fluorophore molecule coupled to each Darpin molecule, was calculated as described below, with $\varepsilon_{280}$(ANBD)= 2100 $M^{-1}$ $cm^{-1}$, $\varepsilon_{500}$(ANBD) = 31800 $M^{-1}$ $cm^{-1}$, both measured with conjugates between IANBD and 2-mercaptoethanol (Renard et al. 2002).

Let P be a protein; B, a mono-conjugate between P and IANBD; Φ, the conjugated form of IANBD; $A_{280}$ and $A_{500}$, the absorbencies of the mixture of P and B that results from the coupling reaction and elimination of the unconjugated fluorophore. Because (i) absorbencies are bilinear functions of molar absorbencies and concentrations, (ii) the molar absorbencies of different chemical groups in a protein molecule are generally additive (Pace et al. 1995), and (iii) proteins generally do not absorb at 500 nm, one can write:

$$y_c = [B]/([B] + [P]) \quad (1)$$

$$y_c^{-1} = (A_{280}/\varepsilon_{280}(P))(A_{500}/\varepsilon_{500}(\Phi))^{-1} - \varepsilon_{280}(\Phi)/\varepsilon_{280}(P) \quad (2)$$

where [B] and [P] are concentrations, and ε is a molar absorbance. There is a corrective term in $y_c^{-1}$, which is constant and comes from the contribution of Φ to $A_{280}$.

### 1.7 Fluorescence measurements and antigen binding

The inventors reasoned for the binding and fluorescence experiments at equilibrium as if the preparations of conjugates were homogeneous, i. e. as if $y_c$ = 1. The binding reactions were conducting by incubating 0.3 μM of conjugate with variable concentrations of the MalE antigen in a volume of 1 mL, for 1 hour in the dark with gentle shaking. They were established in buffer L1, or buffer M1, or in a mixture v:(1-v) of calf serum and buffer M1. The conjugate (or biosensor) B and antigen A form a complex B:A according to the reaction:

$$B + A \leftrightarrow B{:}A \quad (3)$$

At equilibrium, the concentration [B:A] of the complex is given by the equation:

$$[B{:}A] = 0.5\{[B]_0 + [A]_0 + K_d - (([B]_0 + [A]_0 + K_d)^2 - 4\,[B]_0[A]_0)^{1/2}\} \quad (4)$$

where $K_d$ is the dissociation constant between A and B, and $[A]_0$ and $[B]_0$ are the total concentrations of A and B in the reaction, respectively (Renard et al. 2003).

The fluorescence of the IANBD conjugates was excited at 485 nm (5 nm slit width) and its intensity measured at 535 nm (20 nm slit width) with a LS-5B spectrofluorometer (Perkin-Elmer). The signal of MalE alone was measured in an independent experiment and subtracted from the global signal of the binding mixture to give the specific fluorescence intensity $F$ of each conjugate. The intensity $F$ satisfies the following equation:

$$(F - F_0)/F_0 = \Delta F/F_0 = (\Delta F_\infty/F_0)([B{:}A]/[B]_0) \qquad (5)$$

where $F_0$ and $F_\infty$ are the values of $F$ at zero and saturating concentration of A (Renard et al. 2003). The values of $\Delta F_\infty/F_0$, $[B]_0$ and $K_d$ could be determined by fitting equation 5, in which [B:A] is given by equation 4, to the experimental values of $\Delta F/F_0$, measured in a titration experiment. The fittings were performed with the Kaleidagraph software (Synergy Software).

The sensitivity $s$ and relative sensitivity $s_r$ of a conjugate are defined by the following equations, for the low values of $[A]_0$:

$$\Delta F = s[A]_0 \qquad (6)$$

$$\Delta F/F_0 = s_r[A]_0/[B]_0 \qquad (7)$$

$s$ and $s_r$ can be expressed as functions of characteristic parameters of the conjugate:

$$s_r = (\Delta F_\infty/F_0)([B]_0/(K_d + [B]_0)) \qquad (8)$$

$$s = f_b s_r \qquad (9)$$

where $f_b$ is the molar fluorescence of the free conjugate (Renard and Bedouelle 2004). Equation (6) implies that the lower limit of detection $\delta[A]_0$ of the conjugate is linked to the lower limit of measurement of the spectrofluorimeter $\delta F$ by the proportionality factor $s^{-1}$.

### 1.8 Quenching by potasium iodide

The experiments of fluorescence quenching by KI were performed at 25 ˚C in buffer M1, essentially as described above. The Stern-Volmer equation 10 was fitted to the experimental data, where $F$ and $F^0$ are the intensities of fluorescence for the DarpOff7 conjugate, with or without quencher, respectively. The Stern-Volmer constant $K_{SV}$ was used as a fitting parameter.

$$F^0/F = 1 + K_{SV}[KI] \qquad (10)$$

### 1.9 Affinity in solution as determined by competition Biacore

The binding reactions (100 μl) were conducting by incubating 50 nM of DarpOff7 with variable concentrations of MalE in buffer M2 or L2 for 1 hour. It results from the laws of mass action and conservation that:

$$[P] = 0.5\{[P]_0 - [A]_0 - K_d + (([P]_0 - [A]_0 - K_d)^2 + 4\,K_d[P]_0)^{1/2}\} \qquad (11)$$

where $[A]_0$ is the total concentration of MalE in the reaction mixture; $[P]_0$, the total concentration of DarpOff7; and [P], the concentration of free DarpOff7 (Lisova et al. 2007). The concentration of free DarpOff7 was measured by Biacore, essentially as described (Nieba et al. 1996). High densities of MalE (> 2000 Resonance Units, RU) were immobilized on the surface of a streptavidin SA sensor-chip (Biacore). Each reaction mixture was injected in the sensor chip at a flow rate of 25 μL $min^{-1}$. The chip surface was regenerated by injecting 10 μL of a Glycine-HCl solution at pH 3.0 (Biacore) between each run. The experimental data were cleaned up with the Scrubber program (Biologic Software) and analyzed with the Bia-evaluation 2.2.4 program (Biacore) to determine the initial slope *r* of the association curves, which satisfies the equation (Nieba et al. 1996):

$$r = k_{on}R_{max}[P] \quad (12)$$

where $k_{on}$ is the rate constant of association between the free molecules of DarpOff7 and the immobilized molecules of MalE, and $R_{max}$ is the resonance signal which is obtained with a saturating concentration of DarpOff7. The inventors checked that $R_{max}$ was not altered by the regeneration of the chip surface and remained constant. Therefore:

$$r = r_0[P]/[P]_0 \quad (13)$$

where $r_0$ is the value of *r* for $[A]_0 = 0$. The values of $K_d$ and $r_0$ were determined by fitting equation (13), in which [P] is given by equation (11), to the experimental values of *r*.

**1.10 Kinetic measurements by Biacore**

The kinetic measurements were performed at a flow rate of 25 μL $min^{-1}$ with SA sensor chips. A first cell of the sensor chip was used as a reference, i. e. no ligand was immobilized on the corresponding surface. A second cell was loaded with 500 to 1000 resonance units (RU) of bt-MalE. Solutions of the DarpOff7 derivatives at 8 different concentrations (0.15 to 400 nM) were injected during 8 min to monitor association and then buffer alone during the same time for dissociation. The chip surface was regenerated between the runs by injecting 5 to 10 mM NaOH during 1 min. The signal of the buffer alone was subtracted from the raw signals to obtain the protein signals, and then the protein signal on cell 1 was subtracted from the protein signal on cell 2 to obtain the specific signal of interaction. The kinetic data were cleaned up as above and then the kinetic parameters were calculated by a procedure of global fitting, as implemented in the Bia-evaluation 3.0 software (Biacore). For the wild type DarpOff7 (SEQ ID NO: 2) and its cysteine mutants, the inventors applied a simple kinetic model of Langmuir binding to analyze the data. For the preparations of conjugates, the inventors applied a model with two populations of analytes, whose respective proportions corresponded to the coupling yield $y_c$ of the fluorophore.

**EXAMPLE 2. - Results**

**2.1 Design of the conjugates**

The inventors searched for sites to couple the fluorophore to DarpOff7 that satisfied two principles.

- 1) The environment of the coupling residue should change between the free and bound states of DarpOff7, so that the environment of the fluorophore would also change between the free and bound states of the conjugate, after coupling.
- 2) The coupling residue should not be involved in the interaction between DarpOff7 and MalE, so that the fluorophore would not interfere with the interaction between the conjugate and MalE.

The inventors applied these two principles by using the crystal structure of the complex between DarpOff7 and MalE. They identified the residues of DarpOff7 whose solvent accessible surface area (ASA) varied between its free state and its MalE-bound state. They divided this initial set S of residues in three subsets. Subset S1 contained the residues of S in direct contact with MalE. Subset S2 contained the residues of S that were in indirect contact with MalE, through a water molecule. Subset S3 contained the residues of S without any contact, either direct of indirect, with MalE (Table 1, Figure 1).
In figure 1 the positions of the coupling sites in the structure of DapOff7. The ankyrin repeats are represented in alternating light grey and dark grey, with the N-cap on top. Red, residues in direct contact with MalE (subset S1);

green, residues in indirect contact with MalE, through a water molecule (S2); blue, residues whose solvent ASA varies on the binding of MalE and which are in contact with MalE neither directly nor indirectly (S3). The residues of S2 and S3 were targeted for the coupling of IANBD. DarpOff7 is seen from the position of MalE in their complex.

**Table 1.**

| Residue | ΔASA (Å$^2$) | Contact | Set |
|---|---|---|---|
| Arg23 | 9.1 | None | S3 |
| Asn45 | 9.0 | None | S3 |
| Thr46 | 23.9 | HOH15 | S2 |
| Thr48 | 18.6 | MalE | S1 |
| Leu53 | 12.2 | None | S3 |
| Tyr56 | 49.8 | MalE | S1 |
| Asp77 | 9.2 | HOH94 | S2 |
| Val78 | 34.2 | MalE | S1 |
| Phe79 | 116.6 | MalE | S1 |
| Tyr81 | 47.0 | MalE | S1 |
| Leu86 | 26.6 | MalE | S1 |
| Tyr89 | 58.7 | MalE | S1 |
| Trp90 | 97.0 | MalE | S1 |
| Asp110 | 10.1 | MalE | S1 |
| Ser111 | 0.8 | None | S3 |
| Asp112 | 17.9 | None | S3 |
| Met114 | 0.7 | HOH192 | S2 |
| Leu119 | 0.2 | None | S3 |
| Lys122 | 1.6 | HOH29,132 | S2 |
| Trp 123 | 64.3 | MalE | S1 |
| Tyr125 | 13.6 | MalE | S1 |

Table 1 shows the analysis of the interface between DarpOff7 and MalE in the crystal structure of their complex. Column 1, residues of DarpOff7 for which ΔASA ≠ 0. Column 2, variation of ASA between the free and MalE-bound states of DarpOff7 for the residue in column 1. Column 3, molecules in contact with the residue of column 1. Column 4, sub-sets of the residues in column 1: S1, residues in direct contact with MalE; S2, residues in contact with MalE through a water molecule; S3, residues not in contact. The water molecules are numbered according to the PDB file 1SVX. HOH29 and HOH 132 belong to a network of six water molecules (HOH20, 110, 29, 132, 147, 171) that are hydrogen-bonded and located in the interface between DarpOff7 and MalE.

The classifications of the residues were identical when the inventors considered the whole residues or only their side-chains. The inventors targeted the coupling of the fluorophore to the residues of subsets S2 and S3, and rejected those of subsets S1 to avoid affecting the affinity between DarpOff7 and MalE. However, the inventors also rejected residues Asp77 and Leu119 for the following reasons. Asp77 of DarpOff7 is indirectly hydrogen-bonded to Lys202 of MalE through a water molecule (HOH94), and such indirect hydrogen bonds can be energetically important (England et al. 1997). The variation of ASA for Leu119 on MalE binding is very small, 0.2 Å$^2$. The inventors thus selected eight residues of DarpOff7 as potential coupling sites. As a negative control for the design, the inventors chose residue Lys68, which is located on the side of DarpOff7 that is opposite the MalE binding site.

**2.2 Production and oligomeric state of the cysteine mutants**

The eight targeted residues of DarpOff7 and the control residue were changed individually into cysteine by site-directed mutagenesis of the coding gene. The mutant Darpins were produced in the cytoplasm of *E. coli* at 37 °C and purified through their hexahistidine tag. The yield of purified soluble protein varied between 30 mg/L and 100 mg/L of culture. It varied as much between different mutants as between different batches of the same mutant, and was consistent with that reported previously for the wild type DarpOff7 (SEQ ID NO: 2) (Binz et al. 2004).

The introduction of a cysteine residue could lead to intermolecular disulfide bonds. To characterize the oligomeric state of the DatpOff7 mutants, the inventors analyzed the purified preparations by SDS-PAGE after denaturation in the presence or absence of a reducing agent. In reducing conditions, they observed a single protein species with an apparent molecular mass comprised between 16600 and 16900, and consistent with the theoretical mass of a DarpOff7 (wt) monomer, 18272.4. In non-reducing conditions, they observed a second species with an apparent molecular mass comprised between 34100 and 35700, and consistent with the theoretical mass of a dimer, 36542.9. The proportion of monomers in a dimeric state was calculated from the intensities of the protein bands. It varied widely between different mutants, from 3 to 64 % (Table 2).

**Table 2.**

| Mutation | ASA($S_\gamma$) (Å$^2$) | Dimer (%) | $y_c$ | $y_s$ |
|---|---|---|---|---|
| R23C | 17.3 | 69 | 0.98 | 0.60 |
| N45C | 26.8 | 63 | 0.57 | 0.73 |
| T46C | 13.4 | 25 | 0.47 | 0.67 |
| L53C | 3.9 | 8 | 0.99 | 0.67 |
| K68C | 28.0 | 11 | 1.02 | 0.67 |
| S111C | 16.0 | 6 | 0.92 | 0.61 |
| D112C | 19.0 | 7 | 0.76 | 0.67 |
| M114C | 8.0 | 4 | 0.93 | 0.56 |
| K122C | 5.2 | 3 | 1.10 | 0.59 |

Table 2 shows the properties of the cysteine mutants of DarpOff7. Column 1, mutation of DarpOff7. Column 2, ASA of the $S_\gamma$ atom, as measured on a three-dimensional model of the DarpOff7 mutant (see 1. Materials and Methods). Column 3, proportion of polypeptides in a dimeric state, in a purified preparation of the DarpOff7 mutant. Column 4, number of molecules of fluorophore per molecule of DarpOff7 in a purified preparation of the conjugate (coupling yield $y_c$). Column 5, yield of synthesis ($y_s$) for the preparation of a conjugate from a DarpOff7 mutant.

### 2.3 Conjugation and its yield

The inventors submitted the purified preparations of the DarpOff7 mutants to a reaction of reduction before coupling with IANBD, to break open potential intermolecular disulfide bonds and ensure that the mutant cysteine would be in a reactive state.

The products of the coupling reaction were separated from the unreacted fluorophore by chromatography on a nickel ion column. The coupling yield $y_c$ was calculated from the absorbance spectra of the purified reaction product (see 1. Materials and Methods). It was found to be very reproducible, close to 100 % for six of the nine DarpOff7 mutants, and lower for the mutants at positions Asp112 (75 %), Asn45 (57 %) and Thr46 (47 %). The synthesis yield $y_s$ of the coupling procedure, i. e. the proportion of protein molecules that survived the procedure, was close for all the DarpOff7 mutants, 64 $\pm$ 5 % (mean $\pm$ SE, Table 2).

The inventors analyzed the cause for the low yield of coupling in position Asn45, so as to have more homogeneous preparations of conjugates. The inventors have found that the low yield of coupling for DarpOff7(N45C) did not result from a low accessibility of the mutant cysteine to the solvent, since this mutant derivative of DarpOff7 could form an intermolecular disulfide bond efficiently. It also did not result from an irreversible modification of the mutant cysteine since an analysis of a purified preparation of DarpOff7(N45C) by mass spectrometry showed that it contained only two protein species, with molecular masses that were equal to 18275.8 $\pm$ 1.6 and 36548.7 $\pm$ 2.1 and were close to the theoretical masses of the monomeric and dimeric states of DarpOff7(N45C), 18272.4 and 36542.9 respectively. The low yield did not result from an oxidized state of the mutant cysteine because they performed a reducing treatment either before or during the reaction of coupling, with different reducing agents (TCEP or DTT) and at variable concentrations of these agents (from 0.1 to 5 mM), without any change. Moreover, the inventors checked by SDS-PAGE that the protein was in a monomeric state immediately after this treatment. Finally, the low yield did not result from slow coupling kinetics because it was not changed by an increase in temperature (from 30 °C to 40 °C) or the duration of the reaction (from 30 min to overnight). Therefore, the inventors are unable to explain the differences in the yields of coupling.

### 2.4 Fluorescence properties of the conjugates

The fluorescence of the conjugates was excited at 485 nm and recorded at 535 nm. The inventors tested the responsiveness of the DarpOff7 conjugates to the binding of their MalE antigen by measuring the relative variation $\Delta F/F_0 = (F - F_0)/F_0$ in their fluorescence intensity $F$ between their MalE-bound and free states. In a first test, the inventors used a concentration of MalE equal to 2.6 μM, i. e. about 9 times the concentration of conjugate (0.3 μM) and 230 times

the value of the dissociation constant $K_d$ (11 nM) between DarpOff7(wt) and MalE. All the conjugates that the inventors constructed, responded to the binding of MalE, except the DarpOff7(K68ANBD) control. The value of $\Delta F_{2.6\mu M}/F_0$ was comprised between 0.9 and 14.6 for the eight responsive conjugates when the assay was done in the low salt buffer L1 (Table 3).

**Table 3.**

| Residue | Buffer | $f_b$ (FU $\mu M^{-1}$) | $\Delta F_{2.6\mu M}/F_0$ | $\Delta F_\infty/F_0$ | $K_d$ (nM) |
|---|---|---|---|---|---|
| Arg23 | L1 | 341 ± 2 | 0.96 ± 0.03 | 0.96 ± 0.01 | 26 ± 4 |
| Asn45 | L 1 | 232 ± 6 | 14.1 + 0.4 | 14.00 ± 0.07 | 13 ± 2 |
| Thr46 | L1 | 166 ± 2 | 14.5 ± 0.2 | 18.0 ± 0.2 | 546 ± 40 |
| Leu53 | L1 | 276 ± 2 | 2.61 ± 0.05 | 2.91 ± 0.06 | 271 ± 36 |
| Ser111 | L1 | 271 ± 1 | 0.74 ± 0.01 | 0.73 ± 0.01 | 10 ± 3 |
| Asp112 | L1 | 197 ± 2 | 2.09 ± 0.03 | 2.17 ± 0.04 | 121 ± 19 |
| Met114 | L1 | 56 ± 5 | 8.1 ± 0.9 | 8.9 ± 0.2 | 211 ± 30 |
| Lys122 | L1 | 47 ± 1 | 2.21 ± 0.04 | 122 ± 13 | $(4.7 \pm 0.3)x10^6$ |
| Arg23 | M1 | 314 ± 2 | nd | 0.93 ± 0.01 | 18 ± 5 |
| Asn45 | M1 | 266 ± 3 | nd | 8.25 ± 0.06 | 8 ± 2 |
| Thr46 | M1 | 323 ± 4 | nd | 7.92 ± 0.08 | 255 ± 18 |
| Leu53 | M1 | 259 ± 3 | nd | 2.61 ± 0.03 | 104 ± 10 |
| Asn45 | Serum | 452 ± 4 | nd | 2.11 ± 0.01 | 18 ± 2 |

Table 3 shows the properties of DarpOff7 conjugates, as derived from fluorescence experiments. Column 1, residue with which the fluorophore was coupled. Column 3, molar fluorescence $f_b$ of the free conjugate. The total concentration of conjugate was equal to $0.3 y_c$ μM, where the coupling yield $y_c$ is given in Table 2. The entries for $f_b$ and $\Delta F_{2.6\mu M}/F_0$ give the mean value and associated standard error (SE) in at least two experiments. The entries for $\Delta F_\infty/F_0$ and $K_d$ give the value and associated SE in the fitting of Equation 5 to the data points in the titration experiments. The Pearson parameter in these fittings was $R > 0.996$. The $K_d$ value for DarpOff7(wt) was equal to 11 ± 1 nM in buffer L2 and 5.6 ± 0.8 nM in buffer M2, as measured by competition Biacore. Serum, 90 % calf serum; nd, not determined. The SE value on $\Delta F_{2.6\mu M}/F_0$ was calculated through the equation $[SE(\Delta F_{2.6\mu M}/F_0)]^2 = (F_{2,6\mu M}/F_0)^2\{[SE(F_{2,6\mu M})/F_{2,6\mu M}]^2 + [SE(F_0)/F_0]^2\}$.

The values of $\Delta F_{2.6\mu M}/F_0$ varied between conjugates. These variations could come either from different interactions between the fluorescent group and MalE, or from different affinities between the conjugates and MalE. To distinguish between these mechanisms and characterize the properties of the eight conjugates in more details, they determined the relation between the intensity of fluorescence and concentration of MalE for each conjugate by titration experiments in buffer L1 (Figure 2).

In figure 2 the Titration of DarpOff7 conjugates by MalE, monitored by fluorescence. The experiments were performed at 25 °C in buffer M1. The total concentration in DarpOff7, measured by $A_{280}$, was equal to 0.3 μM. The total concentration in the MalE protein is given along the *x* axis. The continuous curves correspond to the fitting of Equation 5 to the experimental values of $\Delta F/F_0$ (see Materials and Methods for details). (Δ) position Arg23; (●) Asn45; (○) Thr46; (▲) Leu53; (♦) Lys68.

The theoretical Equation 5, linking $\Delta F/F_0$ and the concentration of antigen, was fitted to the experimental data with the concentration of functional conjugate, $\Delta F_\infty/F_0$ and $K_d$ as fitting parameters (Table 3). The values of $\Delta F_\infty/F_0$ and $\Delta F_{2.6\mu M}/F_0$ were close for seven of the eight conjugates but differed by 55-fold for DarpOff7(K122ANBD). The values of $K_d$ differed widely between conjugates and were comprised between 10 nM and 4.7 mM. These values were several fold lower than 2.6 μM for the seven conjugates above and therefore these conjugates were saturated by MalE at a concentration of 2.6 μM. In contrast, $K_d$ was 1800-fold higher than 2.6 μM for DarpOff7(K122ANBD). Therefore this conjugate was not saturated by MalE at 2.6 μM, which explained the large difference between its values of $\Delta F_\infty/F_0$ and $\Delta F_{2.6\mu M}/F_0$ (2.2 and 121 respectively). Note that the high value of $\Delta F_\infty/F_0$ for DarpOff7(K122ANBD) was obtained through a long range extrapolation and might be an overestimate.

### 2.5 Fluorescence and salt effects

The quantum yield of fluorophores and the electrostatic interactions between molecules can be salt sensitive. The salt concentration of the buffer could therefore affect the response of the DarpOff7 conjugates at the levels of both their fluorescent group and interaction with MalE. To test these assumptions, the inventors compared the fluorescence properties of four conjugates, at positions Arg23, Asn45, Thr46 and Leu53, by experiments of titration in the low salt buffer L1 and medium salt buffer M1 (Figure 3).

In figure 3 the selectivity and specificity of the fluorescence signal for the DarpOff7(N45ANBD) conjugate. The experimental conditions were as described in figure 2, except for the buffers. The total concentration in antigen, MalE or BSA, is given along the x axis. (●) MalE in buffer L1; (○) MalE in buffer M1; (♦) MalE in 90 % serum; (◊) BSA in buffer M1.

The inventors also compared the properties of interaction between the parental DarpOff7(wt) and MalE in these two buffers by experiments of competition Biacore (Materials and methods).

The inventors found that the value of $K_d$ for DarpOff7(wt) was slightly higher in buffer L1 than in buffer M1, 11 ± 1 nM versus 5.6 ± 0.8 nM, as measured by competition Biacore. The inventors observed the same trend for the $K_d$S of four conjugates as measured by titration experiments, e. g. the value of $K_d$ for DarpOff7(T46ANBD) was higher by 2-fold in buffer L1 (Table 3). These results suggested that the NaCl ions screened unfavorable electrostatic interactions. The values of $F_0$ and $\Delta F_\infty/F_0$ were very close in buffers L1 and M1 for the conjugates at positions Arg23 and Leu53. In contrast, the values of $F_0$ were lower in buffer L1 than in buffer M1 for the conjugates at positions Asn45 and Thr46, and consequently the values of $\Delta F_\infty/F_0$ were higher, up to 2-fold (Table 3). Thus, lower salt concentrations could increase both $K_d$ and $\Delta F\infty/F_0$ for some conjugates.

### 2.6 Selectivity and specificity

The selectivity of a biosensor refers to the extent to which it can recognize a particular analyte in a complex mixture without interference from other components in the mixture (Vessman et al. 2001). The inventors tried to characterize the selectivity of the DarpOff7(N45ANBD) conjugate by performing experiments of titration by the MalE antigen in a complex medium like serum and by comparing these experiments with those performed in the medium salt buffer M1 (Figure 3). The inventors found that the value of $\Delta F_\infty/F_0$ for DarpOff7(N45ANBD) was high in serum, 2.11 ± 0.01, and that this conjugate recognized MalE with close values of $K_d$ in serum and buffer M1 (Table 3). Thus, DarpOff7 (N45ANBD) recognized MalE selectively in serum. However, the value of $F_0$, the fluorescence intensity of the free conjugate, was 1.7 fold higher and the value of $\Delta F_\infty/F_0$ was 3.9 fold lower in serum than in buffer M1. Thus, some components of the serum interfered with the fluorescence properties of DarpOff7(N45ANBD) as measured by the experimental setting (see below).

The experiments to determine the recognition of MalE by DarpOff7(N45ANBD) in serum and buffer M1 showed that this recognition was selective. To further establish the specificity of the recognition, the inventors titrated DarpOff7 (R23ANBD) and DarpOff7(N45ANBD) with bovine serum albumin (BSA) and hen egg white lysozyme in buffer M1. The inventors found that the values of $\Delta F_{2.6\mu M}/F_0$ were much lower for the non-cognate proteins than for MalE, e. g. 40-fold lower for BSA (Figure 3). Therefore, the variation of the $\Delta F/F_0$ signal was indeed specific of MalE, the cognate antigen.

### 2.7 Binding parameters

The experimental conditions of the titration experiments were not appropriate to evaluate nano-molar values of $K_d$ precisely since the concentration of conjugate was micro-molar. The inventors measured the kinetic parameters of interaction between DarpOff7(wt), four of its cysteine mutants, and the four corresponding conjugates on the one hand, and MalE on the other hand by Biacore to obtain more precise values and better understand the mechanisms of variation in these parameters. The kinetics were performed in the presence of DTT for the cysteine mutants and the corresponding controls to prevent their dimerization. The inventors analyzed the kinetic data with the model of a simple bi-molecular reaction (one analyte and one ligand) for DarpOff7(wt) and its cysteine mutants, and calculated the corresponding dissociation constant from the rate constants, i. e. $K_d' = k_{off}/k_{on}$.

The inventors used the model of a three-molecular reaction (two analytes and one ligand) for the conjugates to take the incomplete coupling of some preparations into account (Table 2). The results of the kinetic experiments are reported in Table 4.

**Table 4.**

| Derivative | Buffer | Model | $k_{on1}$ ($10^5$ $M^{-1}$ $s^{-1}$) | $k_{off1}$ ($10^{-3}$ $s^{-1}$) | $K_{d1}$' (nM) | $k_{on2}$ ($10^5$ $M^{-1}$ $s^{-1}$) | $k_{off2}$ ($10^{-3}$ $s^{-1}$) | $_{Kd2}$' (nM) |
|---|---|---|---|---|---|---|---|---|
| WT | M2 | LB | 6.6 | 5.1 | 7.7 | na | na | na |
| WT | L2 | LB | 2.5 | 6.2 | 25.1 | na | na | na |
| WT | M3 | LB | 5.5 | 6.1 | 11.1 | na | na | na |
| R23C | M3 | LB | 4.1 | 5.0 | 12.4 | na | na | na |
| N45C | M3 | LB | 2.9 | 7.4 | 25.7 | na | na | na |

(continued)

| Derivative | Buffer | Model | $k_{on1}$ ($10^5$ $M^{-1}$ $s^{-1}$) | $k_{off1}$ ($10^{-3}$ $s^{-1}$) | $K_{d1}$' (nM) | $k_{on2}$ ($10^5$ $M^{-1}$ $s^{-1}$) | $k_{off2}$ ($10^{-3}$ $s^{-1}$) | $_{Kd2}$' (nM) |
|---|---|---|---|---|---|---|---|---|
| T46C | M3 | LB | 1.7 | 4.1 | 24.7 | na | na | na |
| L53C | M3 | LB | 3.7 | 6.9 | 18.8 | na | na | na |
| R23ANBD | M2 | HA | 1.8 | 11.7 | 63.2 | 4.3 | 2.7 | 6.2 |
| N45ANBD | M2 | HA | 2.1 | 1.8 | 8.9 | 2.4 | 11.3 | 46 |
| T46ANBD | M2 | HA | 0.64 | 26.1 | 408 | 0.18 | 1.8 | 98 |
| L53ANBD | M2 | HA | 2.0 | 60 | 326 | 5.8 | 4.0 | 6.8 |

Table 4 shows the binding parameters of DarpOff7 and derivatives, as determined by Biacore experiments. The Bt-MalE antigen was immobilized on streptavidin SA sensorchips. The association and dissociation rate constants, $k_{on}$ and $k_{off}$, were determined at 25˚C and used to calculate $K_d$'= $k_{off}$/$k_{on}$ (Materials and Methods). The inventors applied a simple kinetic model of Langmuir binding (LB) for DarpOff7(wt) and its cysteine mutants. The inventors applied a model with two populations of analytes (heterogeneous analyte HA) for the preparations of conjugates to take incomplete coupling into account. na, not applicable.

They found that the value of $K_d$, measured at equilibrium in solution by competition Biacore (see above), and the value of $K_d$', deduced from kinetic experiments at the interface between a liquid and a solid phase, were close for DarpOff7(wt) in medium salt buffer (5.6 ± 0.8 nM versus 7.7 nM). The value of $K_d$' for DarpOff7(wt) was higher in low salt buffer than in medium salt buffer. This variation of $K_d$' with the concentration in salt was consistent with that of $K_d$, although larger (3.5-fold versus 2-fold).

It was mainly due to an increase of $k_{on}$ with the concentration in salt and therefore consistent with a long range effect, e. g. the screening of unfavorable electrostatic interactions between DarpOff7(wt) and MalE by the salt. The other kinetics were performed in medium salt buffer. The mutations into Cys had little effect on the values of $k_{off}$, $k_{on}$ and $K_d$'. The effects were the most important for mutations N45C and T46C (2.2 fold) and mainly due to a slower $k_{on}$.

For the conjugates, the value of $K_{d1}$' measured by Biacore was consistent with the value of $K_d$ measured by fluorescence. The value of $K_{d2}$' was close to that of the corresponding cysteine mutant, except for the preparation of DarpOff7(T46ANBD) for which it was 5.5 fold higher. These kinetic experiments were performed in the absence of a reducing agent, therefore the non-coupled molecules of DarpOff7(T46ANBD) could be in a dimeric state and thus altered in their ability to bind MalE. The value of $K_{d1}$' for DarpOff7(N45ANBD) was close to the value of $K_d$' for DarpOff7(wt); it was 8 fold higher for DarpOff7(R23ANBD) and about 50 fold higher for DarpOff7(T46ANBD) and DarpOff7(L53ANBD). The increase in the $K_{d1}$' value of the conjugates relative to the $K_d$' value of the parental DarpOff7(wt) resulted from variations in both $k_{off}$ and $k_{on}$, and the variation of this latter parameter could be important, 10 fold for DarpOff7(T46ANBD).

A comparison between the values of $K_d$' for the cysteine mutants and $K_d$ or $K_{d1}$' for the conjugates showed that the variations in affinity were mainly due to the coupling of the fluorophore and not to the mutation into Cys. The values of $K_d$, determined by titration experiments, were comprised between the corresponding values of $K_{d1}$' and $K_{d2}$', determined by Biacore. This comparison was consistent with $K_d$ being an apparent dissociation constant and describing a mixture of conjugated and unconjugated molecules. However, one should keep in mind that dissociation constants in solution and at the interface between solid and liquid phases are not generally equal (Rich and Myszka 2005).

### 2.8 Mechanism of fluorescence variation

All eight conjugates that the inventors designed as potential biosensors, were sensitive to the binding of MalE, with $\Delta F_{\infty}/F_0 > 0.73$. To test whether these variations of fluorescence resulted from the proximity between the coupling site of the fluorophore and the binding site of MalE, as assumed in the inventors design scheme, they constructed the DarpOff7(K68ANBD) conjugate. Residue Lys68 is located on the side of DarpOff7 that is opposite the binding site of MalE. They did not observe any variation of fluorescence for DarpOff7(K68ANBD) on the binding of MalE. This observation showed that the fluorophore had to be in the neighborhood of the Darpin binding site for the fluorescence to vary.

They used potassium iodide (KI) to explore the physico-chemical mechanism by which the fluorescence intensity of the conjugates varied on antigen binding. First, the inventors checked by an indirect ELISA that KI, up to 250 mM, did not affect the interaction between the parental DarpOff7(wt) and MalE (Materials and methods). They found that the fluorescence of the DarpOff7(N45ANBD) conjugate was quenched by KI, both in its free and MalE-bound states. The quenching varied linearly with the concentration of KI (Figure 4).

In figure 4 the quenching of the DarpOff7(N45ANBD) fluorescence by KI. *F* and $F^0$, fluorescence of the conjugate at 25 ˚C in buffer M1, with and without quencher respectively. (●) Conjugate (1 μM) in the absence of the MalE antigen; (○) conjugate (0.3 μM) in the presence of a saturating concentration of MalE (1.8 μM). The continuous curves were obtained by fitting Equation 10 to the experimental data.

This law of variation indicated that the molecules of fluorophore constituted a homogeneous population and were identically exposed to KI (Lakowicz 1999). It confirmed that the fluorescent group was specifically coupled to the mutant cysteine. The Stern-Volmer constant was higher for the free conjugate than for its complex with the antigen: $K_{sv}$ = 2.92 ± 0.06 $M^{-1}$ *versus 1.06* ± 0,03 $M^{-1}$ (SE in the curve fits of Figure 4). These values indicated a lower accessibility of the fluorophore to KI in the bound state of the conjugate than in its free state. They showed that the fluorescence increase was due to a shielding of the fluorescent group from the solvent by the binding of the antigen, as previously observed for other conjugates with IANBD (Renard et al. 2003). Thus the mechanism of fluorescence variation was general and consistent with the rules of design.

### 2.9 Mechanism of fluorescence variation in serum

The profiles of titration of DarpOff7(N45ANBD) by MalE were different in calf serum and in a defined buffer (Figure 3). In particular, the inventors observed that the value of $F_\infty$ was lower and that of $F_0$ higher in serum. To better understand these differences, the inventors measured the variations of the $F_0$ and $F_{2.6\mu M}$ parameters as functions of the concentration in serum (Figure 5).

In figure 5 the effect of the concentration in serum on the fluorescence signals for the DarpOff7(N45ANBD) conjugate. The experiments were performed in a mixture (v : 1 - v) of serum and buffer M1. The total concentration of MalE was equal to 2.6 μM and thus saturating. The other experimental conditions were as described in figure 2. (○) $F_0$; (●) $F_{2.6\mu M}$; FU, arbitrary units of fluorescence. The continuous curves were obtained by fitting a linear model of attenuation to the experimental values of $F_{2.6\mu M}$, and a mixed model of association and linear attenuation to the values of $F_0$.

They observed that the value of $F_{2.6\mu M}$ for DarpOff7(N45ANBD) decreased linearly with the concentration in serum. As expected, the absorbance of the serum alone increased linearly with its concentration, in agreement with the Beer-Lambert law, at both 485 nm and 535 nm, which were the wavelengths of fluorescence excitation and emission in the experiments. Therefore, the absorption of the excitation and emission lights by serum could account for the variation of $F_{2.6\mu M}$. Surprisingly, $F_0$ increased with the concentration in serum, up to 40 % (v/v) of serum, and then decreased slowly. The initial increase could result from the interaction between the DarpOff7 conjugate and molecules of the serum, and the subsequent decrease from the absorbance of the serum, as observed for $F_{2.6\mu M}$.

### 2.10 Rules of design and their efficiency

The inventors have developed and validated a method to choose coupling sites for fluorophores in a Darpin and transform it into a reagentless fluorescent biosensor. The method is based on the crystallographic coordinates of the complex between the Darpin and its antigen, and it does not involve any knowledge on their energetic interface. Two criteria were applied: (1) the solvent ASA (accessible surface area) of the target residue should vary between the free and bound states of the Darpin; (2) the target residue should not be in contact with the antigen. The first rule was based on the assumption that the fluorescence variation of the conjugate upon antigen binding is due to a change in the environment of the fluorescent group. The second one aimed at avoiding residues that contribute to the energy of interaction between the Darpin and its antigen.

The inventors applied this method to the complex between DarpOff7 and MalE, and thus selected eight coupling residues in DarpOff7. Each of them gave a conjugate that could detect the binding of MalE with a value $\Delta F_\infty/F_0$ > 0.73. Three conjugates had affinities close to that of DarpOff7(wt) ($\Delta\Delta G$ ≤ 0.5 kcal $mol^{-1}$). The most promising conjugate, DarpOff7(N45ANBD), had a value $\Delta F_\infty/F_0$ = 14.0 ± 0.1 and an affinity nearly identical to that of DarpOff7(wt) ($\Delta\Delta G$ = 0.1 ± 0.01 kcal $mol^{-1}$_). Experiments of fluorescence quenching by KI with the DarpOff7(N45ANBD) conjugate showed that the mechanism of fluorescence variation was consistent with the rules of design and general.

The conjugates that were constructed from the three residues that were in indirect contact with the antigen (Thr46, Met114 and Lys122, belonging to subset S2), had the lowest values of $F_0$ and the highest values of $\Delta F_\infty/F_0$. Residues Thr46 and Met114 make indirect contacts with MalE through a single and isolated water molecule (HOH15 and HOH192 respectively). Lys122 makes indirect contacts with MalE through two water molecules (HOH29 and HOH132) which in turn belong to a network of six water molecules, linked by hydrogen bonds. The corresponding conjugate DarpOff7 (K122ANBD) had an exceptionally high value of $\Delta F_\infty/F_0$. The low $F_0$ values suggested that the fluorescent group was highly exposed to the solvent in the free state of these conjugates. The positions of the water molecules and high $\Delta F_\infty/F_0$ values suggested that the fluorescent group displaced water molecules in the interface between DarpOff7 and MalE in the bound state of these conjugates, and was at least partially buried in this interface. Consistently, the affinities between the three corresponding conjugates and MalE were also much decreased. Residue Asn45, which belonged to the S3 subset, is adjacent to residue Thr46, which belonged to the S2 subset. It is farther from the interface between DarpOff7 and MalE than Thr46. The corresponding conjugate DarpOff7(N45ANBD) had a very high value $\Delta F_\infty/F_0$ = 14.0 ± 0.1 and an unchanged affinity relative to DarpOff7(wt). Its fluorescent group might have replaced HOH15, as the DarpOff7(T46ANBD) one, but without inserting itself as much in the interface. The high values of $\Delta F_\infty/F_0$ that the inventors obtained for some conjugates, showed that the use of the IANBD ester as a fluorophore did not limit the extent of the fluorescence response a priori.

### 2.11 Production and dimerization of the Cys mutants

Some mutant derivatives of DarpOff7, carrying a Cys mutation, formed covalent homodimers through an intermolecular disulfide bond. The relative proportions of monomers and homodimers in the protein preparations varied with the position of the mutation. The inventors assumed that this dimerization occurred during the cellular extraction and purification of proteins since disulfide bonds do not form in the reducing medium of the cytoplasm. The inventors modeled the three-dimensional structure of the mutant DarpOff7 molecules and calculated the solvent ASA of the mutant cysteines (Table 2). As expected, a low accessibility of the $S_\gamma$ atom to the solvent disfavored the formation of a disulfide bond (e. g. at positions Leu53, Met114 and Lys122) whereas a high accessibility favored it (e. g. at positions Arg23 and Asn45) but was not sufficient (e. g. at positions Lys68, Asp112C and Ser111). Likely, the geometrical relationships that are necessary to form a disulfide bond, were not satisfied for these three last mutations (Sowdhamini et al. 1989). Nevertheless, the results demonstrated the possibility of linking two Darpin molecules together through a disulfide bound, which could be used to design bivalent or bifunctionnal Darpin dimers.

### 2.12 Impact of the fluorescent group on antigen binding

The $\Delta F_\infty/F_0$ and $K_d$ parameters were obtained by fitting Equation 5 to titration data. This equation describes the association of homogeneous preparations of protein and antigen. However, MalE was in contact with different DarpOff7 species for the conjugates with a coupling yield < 100 %, i. e. the conjugated species, the cysteine mutant in a monomeric unconjugated form, and the mutant in a homodimeric form. The value of $\Delta F_\infty/F_0$, which is a relative, dimensionless parameter, was not affected by the coupling yield, provided that the coupling was homogeneous. The inventors have shown that such was the case for the DarpOff7(N45ANBD) conjugate in the experiments of fluorescence quenching by KI (see Results). Moreover, the real value of $K_d$ for the interaction between the conjugated species and MalE was necessarily lower than the apparent value of $K_d$ that they obtained with Equation 5 since the concentration of antigen that was available to the conjugate, was lower than or equal to the total concentration. Therefore, the values of $\Delta F_\infty/F_0$ that they report in Table 3, are correct value despite the approximation and the values of $K_d$ are over-estimation, i. e. the real affinities of the conjugates for MalE were higher or equal to the apparent affinities that they found. The value of $K_d$ for the DarpOff7(N45ANBD) conjugate in the titration experiments was compatible with its $K_{d1}$' value in the Biacore experiments. Moreover, the $K_{d2}$' value for this conjugate was consistent with the $K_D$ value for the unconjugated cysteine mutant in the Biacore experiments (Tables 3 and 4). These comparisons indicated that the parameters that the inventors determined to characterize this conjugate, were reliable.

The inventors have shown that the $K_d$' value, measured for four of the cysteine mutants, were only 1.1 to 2.3 fold higher than the $K_d$' value for DarpOff7(wt). Therefore, the variations of $K_d$' that they observed for the conjugates at positions 23, 46 and 53, were mainly due to the presence of the fluorescent group, which affected the interaction between DarpOff7 and MalE.

### 2.13 Classification of the conjugates

The conjugates of DarpOff7 had a wide diversity of values for $\Delta F_\infty/F_0$ and $K_d$. The inventors classified them according to their sensitivity, a parameter which is used to characterize any measuring instrument. This sensitivity can take two forms for a RF biosensor, a relative sensitivity $s_r$ and an absolute sensitivity $s$.

The relative sensitivity $s_r$ relates the relative variation of the fluorescence signal $\Delta F/F_0$ to the relative concentration of antigen $[A]_0/[B]_0$ for the low values, where $[A]_0$ and $[B]_0$ are the total concentrations of antigen and conjugate, respectively, in the measuring reaction (Equation 7 in **1. Materials and Methods**). $s_r$ is an intrinsic dimensionless parameter. Its value does not depend on the spectrofluorometer or its set up, and should remain constant between experiments, instruments and laboratories. The value of $s_r$ depends on the values of $[B]_0$ and $K_d$ according to a saturation law and its maximal value is equal to $\Delta F_\infty/F_0$ (Equation 8).

The absolute sensitivity $s$ relates $\Delta F$ and $[A]_0$ for the low values and is equal to $f_b s_r$, where $f_b$ is the molar fluorescence of the free conjugate (Equations 6 and 9). The $s^{-1}$ parameter relates the lower limit of detection $\delta[A]_0$ for the conjugate to the lower limit of measurement $\delta F$ for the spectrofluorometer.

The inventors calculated the variations of $s_r$ and $s^{-1}$ for each conjugate as a function of $[B]_0$ in the low salt buffer L1 (Figures 6 and 7).

In figure 6 the ranking of the DarpOff7 conjugates according to their relative sensitivities $s_r$ at 25 °C in buffer L1. The $s_r$ parameter relates the relative variation of fluorescence intensity $\Delta F/F_0$ and the relative concentration of antigen

$[A]_0/[B]_0$ for the low values of $[A]_0$, where $[A]_0$ and $[B]_0$ are the total concentration of antigen and conjugate in the binding reaction, respectively (Equations 7 and 8). (■) position Arg23; (●) Asn45; (○) Thr46; (▲) Leu53; (□) Ser111; (Δ) Asp112; (V) Met114; (♦) Lys122.

In figure 7 the Ranking of the DarpOff7 conjugates according to their lower limit of detection at 25 °C in buffer L1. The $s^{-1}$ parameter gives the lower concentration of antigen $[A]_0$ that can be detected by a conjugate when the lower variation of fluorescence intensity that can be detected by the spectrofluorometer, is equal to 1 FU. (■) position Arg23; (●) Asn45; (○) Thr46; (▲) Leu53; (□) Ser111; (△) Asp 112; (▼) Met 114.

These variations showed that the classification of the conjugates varied as a function of $[B]_0$. For $s_r$ and with $[B]_0$ = 0.3 μM, i. e. the concentration at which the inventors performed there experiments, the coupling positions ranked in the following order: Asn45 > Thr46 > Met114 > Leu53 ≈ Asp112 > Arg23 > Ser111. For $s^{-1}$ and $[B]_0$ = 0.3 μM, the coupling positions ranked in the following order: Asn45 < Thr46 < Leu53 < Arg23 ≈ Asp112 ≈ Met114 < Ser111. DarpOff7 (N45ANBD) at 0.3 μM had a value $s^{-1}$ = 0.32 nM $FU^{-1}$ and therefore a lower limit of detection $\delta[A]_0$ = 0.32 nM in the experiments since the Perkin-Elmer SF5B spectrofluorometer could detect a variation of fluorescence δF = 1 FU.

## 2.14 Coupling sites in the absence of structural data

To be useful, the approach that the inventors have developed and validated with DarpOff7, should be generalized to Darpins of unknown structures because structural data are not always available.

Each ankyrin repeat (AR) module, except the specialised N-cap and C-cap modules, comprises 33 residues. The positions that have been randomized to construct the combinatorial library of Darpins, correspond to potential residues of interaction with the antigen and they occupy positions 2, 3, 5, 13, 14, 26 and 33 of each module. The inventors observed that the architecture of a Darpin is such that residues that occupy equivalent positions in the sequences of two adjacent modules, are neighbors in the three-dimensional structure.

The comparative analysis of the sequence and structure of DarpOff7 showed that the central AR2 module provided the majority of the direct contacts with the MalE antigen (subset S1, Table 1 and Figure 8).

In figure 8 the relative positions of the coupling sites in the ankyrin repeats. Positions 2, 3, 5, 13, 14, 26 and 33 in each ankyrin repeat, and positions 43 in the N-cap module were randomized in the initial combinatorial library (Binz et al. 2004). The figure gives the positions of the corresponding residues in the sequence of DarpOff7, the positions of the structurally equivalent residues in the N-cap and C-cap, and the positions of the residues in subsets S1, S2 and S3. The residues of S2 and S3 that were targeted for the coupling of a fluorophore, are generally structurally equivalent to a residue in S1 but in an adjacent ankyrin repeat.

Six among the ten residues of DarpOff7 that were not in direct contact with MalE but whose solvent ASA varied between its free and bound states (subsets S2 and S3), occupied positions of the AR1 and AR3 modules that were randomized and equivalent to positions of direct contact. For example, positions 45 and 111 are equivalent to position 78; 46 and 112 to 79; 114 to 81 and 122 to 89 (Figures 1 and 8). Several of these latter positions gave cysteine mutants or conjugates whose affinity for MalE was similar to that of DarpOff7(wt).

This analysis suggested to the inventors a strategy to construct RF biosensors from Darpins whose three-dimensional structure is unknown. The first step would consist in considering the set $S_R$ of the randomized positions and identifying the positions of $S_R$ that are important for the energy of interaction with the antigen (subset $S_{RE}$) and those that are unimportant (subset $S_{RNE}$), by an alanine or cysteine scanning mutagenesis. The second step would consist in targeting the positions of $S_{RNE}$ that are equivalent to positions of $S_{RE}$ and in an adjacent module, for the coupling of a fluorophore. The equivalent positions of the N-cap and C-cap modules could also be targeted. Alternatively, a strategy analogous to the one that they have validated with antibody fragments, could be used (Renard et al. 2003). The fluorophore could be coupled to the positions of the Darpin that are adjacent to the positions of $S_{RE}$ in the sequence but unimportant for both its interaction with the antigen and folding.

The inventors have developed a method to construct reagentless fluorescent (RF) biosensors from Darpins when the crystal structure of the complex with the antigen is available. This method could be applied to any antigen binding protein in the same conditions. The inventors have validated the method by constructing eight conjugates between the IANBD fluorophore and DarpOff7, a Darpin that is directed against the MalE protein from *E. coli.* The inventors ranked the conjugates according to their relative sensitivity $s_r$ and their lower limit of detection (proportional to $s^{-1}$) and showed that this ranking depended on the concentration in conjugate. One of the conjugates had values $s_r$ > 6 and $s^{-1}$ < 0.7 nM for a concentration of the conjugate equal to 10 nM, and $s_r$ > 12 and $s^{-1}$ < 0.35 nM for a concentration of the conjugate equal to 100 nM. It could function in a complex mixture like serum and the mechanism of its fluorescence variation was general. An analysis of the results on DarpOff7 allowed the inventors to propose a method to construct RF biosensors from Darpins whose structure is unknown. The yields of production of DarpOff7 and its cysteine mutants, and the yields of synthesis of the conjugates with the IANBD fluorophore were much higher than those for scFv fragments of antibodies. The sensitivities of the conjugates from DarpOff7 were generally several fold higher than those from scFv fragments. Therefore, the Darpins, which are very stable proteins, constitute a promising alternative to antibody fragments for the construction and the multiple applications of reagentless fluorescent biosensors, directed against any protein antigen.

## REFERENCES

Altschuh, D., Oncul, S., and Demchenko, A.P. 2006. Fluorescence sensing of intermolecular interactions and development of direct molecular biosensors. J Mol Recognit 19: 459-477.

Amstutz, P., Binz, H.K., Parizek, P., Stumpp, M.T., Kohl, A., Grutter, M.G., Forrer, P., and Pluckthun, A. 2005. Intracellular kinase inhibitors selected from combinatorial libraries of designed ankyrin repeat proteins. J Biol Chem 280: 24715-24722.

Binz, H.K., Amstutz, P., Kohl, A., Stumpp, M.T., Briand, C., Forrer, P., Grutter, M.G., and Pluckthun, A. 2004. High-affinity binders selected from designed ankyrin repeat protein libraries. Nat Biotechnol 22: 575-582.

Binz, H.K., Amstutz, P., and Pluckthun, A. 2005. Engineering novel binding proteins from nonimmunoglobulin domains. Nat Biotechnol 23: 1257-1268.

Binz, H.K., Stumpp, M.T., Forrer, P., Amstutz, P., and Pluckthun, A. 2003. Designing repeat proteins: well-expressed, soluble and stable proteins from combinatorial libraries of consensus ankyrin repeat proteins. J Mol Biol 332: 489-503.

Bullock, W.O., Fernandez, J.M., and Short, J.M. 1987. XL1-Blue: a high efficiency plasmid transforming recA Escherichia coli strain with beta-galactosidase selection. BioTechniques 5: 376-379.

de Lorimier, R.M., Smith, J.J., Dwyer, M.A., Looger, L.L., Sali, K.M., Paavola, C.D., Rizk, S.S., Sadigov, S., Conrad, D.W., Loew, L., et al. 2002. Construction of a fluorescent biosensor family. Protein Sci 11: 2655-2675.

de Lorimier, R.M., Tian, Y., and Hellinga, H.W. 2006. Binding and signaling of surface-immobilized reagentless fluorescent biosensors derived from periplasmic binding proteins. Protein Sci 15: 1936-1944.

England, P., Bregegere, F., and Bedouelle, H. 1997. Energetic and kinetic contributions of contact residues of antibody D1.3 in the interaction with lysozyme. Biochemistry 36: 164-172.

Foote, J., and Winter, G. 1992. Antibody framework residues affecting the conformation of the hypervariable loops. J Mol Biol 224: 487-499.

Griffiths, D., and Hall, G. 1993. Biosensors--what real progress is being made? Trends Biotechnol 11: 122-130.

Harlow, E., and Lane, D. 1988. Antibodies: a laboratory manual. Cold Spring Harbor Laboratory, Cold Spring Harbor, NY, pp. xiii, 726 p.

Huber, T., Steiner, D., Rothlisberger, D., and Pluckthun, A. 2007. In vitro selection and characterization of DARPins and Fab fragments for the co-crystallization of membrane proteins: The Na(+)-citrate symporter CitS as an example. J Struct Biol 159: 206-221.

Kawe, M., Forrer, P., Amstutz, P., and Pluckthun, A. 2006. Isolation of intracellular proteinase inhibitors derived from designed ankyrin repeat proteins by genetic screening. J. Biol. Chem. 281: 40252-40263. **P5**

Jespers, L., Bonnert, T.P., and Winter, G. 2004. Selection of optical biosensors from chemisynthetic antibody libraries. Protein Eng Des Sel 17: 709-713.

Kohl, A., Binz, H.K., Forrer, P., Stumpp, M.T., Pluckthun, A., and Grutter, M.G. 2003. Designed to be stable: crystal structure of a consensus ankyrin repeat protein. Proc Natl Acad Sci U S A 100: 1700-1705.

Lakowicz, J.R. 1999. Principles of fluorescent spectroscopy, 2nd ed. Kluwer Academic/Plenum, New York.

Li, J., Mahajan, A., and Tsai, M.D. 2006. Ankyrin repeat: a unique motif mediating protein-protein interactions. Biochemistry 45: 15168-15178.

Lisova, O., Hardy, F., Petit, V., and Bedouelle, H. 2007. Mapping to completeness and transplantation of a group-specific, discontinuous, neutralizing epitope in the envelope protein of dengue virus. J Gen Virol 88: 2387-2397.

Lowe, C.R. 1984. Biosensors. Trends Biotechnol 2: 59-65.

Mathonet, P., and Fastrez, J. 2004. Engineering of non-natural receptors. Curr Opin Struct Biol 14: 505-511.

Morgan, C.L., Newman, D.J., and Price, C.P. 1996. Immunosensors: technology and opportunities in laboratory medicine. Clin Chem 42: 193-209.

Mosavi, L.K., Cammett, T.J., Desrosiers, D.C., and Peng, Z.Y. 2004. The ankyrin repeat as molecular architecture for protein recognition. Protein Sci 13: 1435-1448.

Mosavi, L.K., Minor, D.L., Jr., and Peng, Z.Y. 2002. Consensus-derived structural determinants of the ankyrin repeat motif. Proc Natl Acad Sci U S A 99: 16029-16034.

Nieba, L., Krebber, A., and Pluckthun, A. 1996. Competition BIAcore for measuring true affinities: large differences from values determined from binding kinetics. Anal Biochem 234: 155-165.

Pace, C.N., Vajdos, F., Fee, L., Grimsley, G., and Gray, T. 1995. How to measure and predict the molar absorption coefficient of a protein. Protein Sci 4: 2411-2423.

Renard, M., and Bedouelle, H. 2004. Improving the sensitivity and dynamic range of reagentless fluorescent immunosensors by knowledge-based design. Biochemistry 43: 15453-15462.
Renard, M., Belkadi, L., and Bedouelle, H. 2003. Deriving topological constraints from functional data for the design of reagentless fluorescent immunosensors. J Mol Biol 326: 167-175.
Renard, M., Belkadi, L., Hugo, N., England, P., Altschuh, D., and Bedouelle, H. 2002. Knowledge-based design of reagentless fluorescent biosensors from recombinant antibodies. J Mol Biol 318: 429-442.
Rich, R.L., and Myszka, D.G. 2005. Survey of the year 2004 commercial optical biosensor literature. J Mol Recognit 18: 431-478.
Rondard, P., and Bedouelle, H. 1998. A mutational approach shows similar mechanisms of recognition for the isolated and integrated versions of a protein epitope. J Biol Chem 273: 34753-34759.
Smith, J.J., Conrad, D.W., Cuneo, M.J., and Hellinga, H.W. 2005. Orthogonal site-specific protein modification by engineering reversible thiol protection mechanisms. Protein Sci 14: 64-73.
Smith, P.A., Tripp, B.C., DiBlasio-Smith, E.A., Lu, Z., LaVallie, E.R., and McCoy, J.M. 1998. A plasmid expression system for quantitative in vivo biotinylation of thioredoxin fusion proteins in Escherichia coli. Nucleic Acids Res 26: 1414-1420.
Sowdhamini, R., Srinivasan, N., Shoichet, B., Santi, D.V., Ramakrishnan, C., and Balaram, P. 1989. Stereochemical modeling of disulfide bridges. Criteria for introduction into proteins by site-directed mutagenesis. Protein Eng 3: 95-103.
Tatutsova and Madden, FEMS Microbiol Lett., 1999, 174, 247-250
Thevenot, D.R., Toth, K., Durst, R.A., and Wilson, G.S. 2001. Electrochemical biosensors: recommended definitions and classification. Biosens Bioelectron 16: 121-131.
Vessman, J., Stefan, R.I., Van Staden, J.F., Danzer, K., Lindner, W., Bums, D.T., Fajgelj, A., and Muller, H. 2001. Selectivity in analytical chemistry - (IUPAC Recommendations 2001). Pure and Applied Chemistry 73: 1381-1386.
Vo-Dinh, T., and Kasili, P. 2005. Fiber-optic nanosensors for single-cell monitoring. Anal Bioanal Chem 382: 918-925.
Vriend, G. 1990. WHAT IF: a molecular modeling and drug design program. J Mol Graph 8: 52-56, 29.
Zahnd, C., Amstutz, P., and Pluckthun, A. 2007. Ribosome display: selecting and evolving proteins in vitro that specifically bind to a target. Nat Methods 4: 269-279.
Zahnd, C., Pecorari, F., Straumann, N., Wyler, E., and Pluckthun, A. 2006. Selection and characterization of Her2 binding-designed ankyrin repeat proteins. JBiol Chem 281: 35167-35175.
Zhu, H., and Snyder, M. 2003. Protein chip technology. Curr Opin Chem Biol 7: 55-63.

## Claims

1. A reagentless peptide biosensor for at least one ligand, comprising:

   at least one ankyrin repeat module;
   at least one cysteine residue coupled to a fluorophore.

2. The biosensor of claim 1, wherein the cysteine residue is present at a position of said biosensor whose solvent accessible surface area is altered when said biosensor binds to said at least one ligand but which does not directly interact therewith.

3. The biosensor of claim 1 or 2, wherein at least one ankyrin repeat of said at least one ankyrin repeat module consists of SEQ ID NO: 3 or SEQ ID NO: 8, or a sequence of at least 60% similarity therewith.

4. The biosensor of claim 3, wherein said fluorophore is coupled to one residue of SEQ ID NO: 3 or SEQ ID NO: 8 selected from:

(i) residues 2, 3, 5, 13, 14, 26 and 33; or
(ii) residues 1, 4, 6, 12, 15, 25, 27, 32.

5. The biosensor of claim 1 to 4, comprising at least an N-terminal capping ankyrin repeat and/or a C-terminal capping ankyrin repeat.

6. The biosensor of claim 5, wherein said N-terminal capping ankyrin repeat consists of SEQ ID NO: 4 and said C-terminal capping ankyrin repeat consists of SEQ ID NO: 5.

7. The biosensor of claim 1 to 6, wherein said at least one cysteine residue is either present in said biosensor or is substituted for another suitable residue whose solvent accessible surface area alters when said biosensor binds to said ligand but which does not directly interact therewith.

8. The biosensor of claim 1 to 7, wherein said at least one residue forms an indirect contact with said ligand via at least one water molecule.

9. The biosensor of any one of claims 1 to 7, wherein said at least one residue does not contact said ligand.

10. The biosensor of any one of claims 1 to 9, comprising more than one ankyrin repeat module.

11. The biosensor of claim 10, wherein a residue in a second domain corresponding to a contacting residue in a first domain, is coupled to a fluorophore.

12. The biosensor as claimed in any one of claims 1 to 11, wherein said fluorophore is selected from the group consisting of: IANBD, CNBD, acrylodan, 5-iodoacetamidofluorescein or a fluorophore having an aliphatic chain of 1 to 6 carbon atoms.

13. The biosensor as claimed in any one of claims 1 to 12, wherein said biosensor is in soluble form.

14. The biosensor as claimed in any one of claims 1 to 13, wherein said biosensor is immobilized on a suitable solid support.

15. The biosensor as claimed in any one of claims 1 to 9, or claims 12 to 14, wherein said biosensor consists of SEQ ID NO: 2 in which at least one of residues 23, 45, 46, 53, 111, 112, 114, 122, 123 and 125 have been substituted with a cysteine residue and coupled to said fluorophore.

16. A protein-based chip, **characterized in that** it consists of a solid support on which at least one biosensor as claimed in any one of claims 1 to 15 is immobilized.

17. A solution comprising at least one biosensor as claimed in any one of claims 1 to 15.

18. An optical fibre comprising at a first end thereof at least one biosensor as claimed in any one of claims 1 to 15 and comprising at a second end thereof means to attach said optical fibre to a device configured to receive an interpret the output of said at least one biosensor.

19. A method for producing biosensors as claimed in any one of claims 1 to 18, **characterized in that** it comprises the following steps:

(a) selecting at least one residue of the biosensor by searching for the residues which have a solvent accessible surface area (ASA) which is modified by the binding of said at least one ligand, when use is made of spheres of increasing radius of 1.4 to 30 A, for the molecule of said solvent; and which (i) are in contact with said ligand via a water molecule, or (ii) do not contact said ligand;
(b) mutating by site-directed mutagenesis at least one of the residues selected in (a) to a Cys residue when said residue is not naturally a Cys residue, and
(c) coupling the Sγ atom of at least one Cys residue obtained in (a) or in (b) to a fluorophore.

**20.** The preparation method as claimed in claim 19, **characterized in that,** prior to step (a), it comprises a step of modelling the biosensor and/or the ligand and/or the biosensor-ligand complex.

**21.** A method for preparing biosensors as claimed in any one of claims 1 to 18, **characterized in that** it comprises the following steps:

(a1) identifying the binding site of the biosensor by mutagenesis of the set, or of a subset, of the residues of the biosensor, and determining the variations in the parameters of interaction with the ligand ($K_d$, $k_{on}$, $k_{off}$) which are due to each mutation or to limited groups of mutations;
(b1) selecting the Cys residues, or the residues to be mutated to cysteine, from the residues of the biosensor which are located in the proximity of the residues of the active site along the sequence;
(c1) mutating by site-directed mutagenesis at least one of the residues selected in (b1) to a Cys residue when said residue is not naturally a Cys residue; and
(d1) coupling the Sγ atom of at least one Cys residue obtained in (b1) or in (c1) to a fluorophore.

**22.** The preparation method of claim 21, wherein in step (b1) the selected residues are adjacent to the residues of the binding site along the peptide backbone (residues -1 and +1).

**23.** A method for preparing biosensors as claimed in any one of claims 1 to 18 which comprises at least two ankyrin repeat modules, **characterized in that** it comprises the following steps:

(a2) identifying the binding site of a first ankyrin repeat module by scanning mutagenesis of the set or of a subset of the residues of said first ankyrin repeat module, and determining the variations in the parameters of interaction with the ligand ($K_D$, $k_{on}$, $k_{off}$) which are due to each mutation or to limited groups of mutations;
(b2) selecting the Cys residues, or the residues to be mutated into cysteine, from the residues of a second ankyrin repeat module which are equivalent to the residues of the binding site or are located in the proximity of the residues of the binding site of said first ankyrin repeat module;
(c2) mutating by site-directed mutagenesis at least one of the residues selected in (b2) to a Cys residue when said residue is not naturally a Cys residue; and
(d2) coupling the Sγ atom of at least one Cys residue obtained in (b2) or in (c2) to a fluorophore.

**24.** The preparation method as claimed in any one of claims 19 to 23, **characterized in that,** prior to step (a), (a1) or (a2), the nonessential Cys residues of the biosensor are substituted with Ser or Ala residues by site-directed mutagenesis.

**25.** The preparation method as claimed in any one of claims 19 to 24, **characterized in that,** in step (c), (d1) or (d2), said fluorophore is selected from the group consisting of: IANBD, CNBD, acrylodan, 5-iodoacetamidofluorescein or a fluorophore having an aliphatic chain of 1 to 6 carbon atoms.

**26.** The preparation method as claimed in any one of claims 19 to 25, **characterized in that,** prior to step (c), (d1) or (d2), the mutated biosensor obtained in step (b), (c1) or (c2) is subjected to a controlled reduction.

**27.** The preparation method as claimed in any one of claims 19 to 26, **characterized in that,** after step (c), (d1) or (d2), it comprises an additional step (d), (e1) or (e2) for purifying the biosensor.

**28.** The preparation method as claimed in claim 27, **characterized in that,** after step (d), (e1) or (e2), it comprises an additional step for (i) measuring the equilibrium constant ($K_D$ or $K'_D$) between said purified biosensor and said at least one ligand, or the dissociation ($K_{off}$) and association ($k_{on}$) rate constants for said biosenor and said at least one ligand; and (ii) measuring the fluorescence variation of said biosensor on ligand binding.

**29.** The preparation method as claimed in any one of claims 19 to 28, **characterized in that,** after step (c), (d1) or (d2) or step (d), (e1) or (e2), it comprises an additional step for immobilizing the biosensor on a solid support.

**30.** The use *in vitro,* of a biosensor as claimed in any one of claims 1 to 18, for applications comprising detecting, assaying and locating said at least one ligand.

**31.** The use of biosensors as claimed in any one of claims 1 to 18, for screening protein libraries.

**32.** The use of biosensors as claimed in any one of claims 1 to 18, for sorting molecules.

**33.** The use of the biosensors as claimed in any one of claims 1 to 18, for sorting cells.

**34.** The use of the biosensors as claimed in any one of claims 1 to 18, for producing protein-based chips.

**35.** A reagent for detecting, assaying or locating ligands, **characterized in that** it includes at least one biosensor as claimed in any one of claims 1 to 18.

**36.** A method for detecting, assaying or locating a ligand in a heterogeneous sample, **characterized in that** it comprises bringing said heterogeneous sample into contact with at least one reagent as claimed in claim 35.

**37.** A kit for detecting, assaying or locating ligands, **characterized in that** it includes at least one reagent as claimed in claim 35.

**38.** A kit for screening for inhibitors of the ligand/receptor interaction, **characterized in that** it includes at least one reagent as claimed in claim 35.

**39.** A reagentless peptide biosensor for at least one ligand, wherein said biosensor comprises at least two ankyrin repeat modules and each of said ankyrin repeat modules comprises at least two cysteine residues, and wherein a fluorophore is attached to a first cysteine residue in each of said ankyrin repeat modules, and wherein each of said ankyrin repeat modules is linked to at least one other of said ankyrin repeat modules via a disulfide bond between a second cysteine residue in each of said ankyrin repeat modules.

**40.** The reagentless biosensor of claim 39, wherein said at least two ankyrin repeat modules are homologous.

**41.** The reagentless biosensor of claim 39, wherein said at least two ankyrin repeat modules are heterologous.

**42.** The reagentless biosensor of claim 41, wherein each of said heterologous ankyrin repeat modules comprise a different fluorophore.

**43.** The biosensor of any one of claims 39 to 42, wherein each said first cysteine residue is present at a position of each said ankyrin repeat module whose solvent accessible surface area is altered when said biosensor binds to said at least one ligand but which does not directly interact therewith.

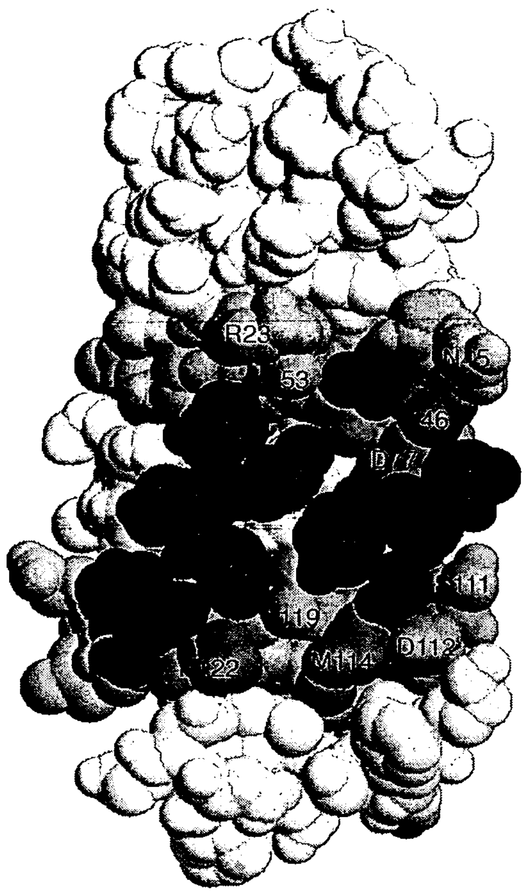


Figure 1

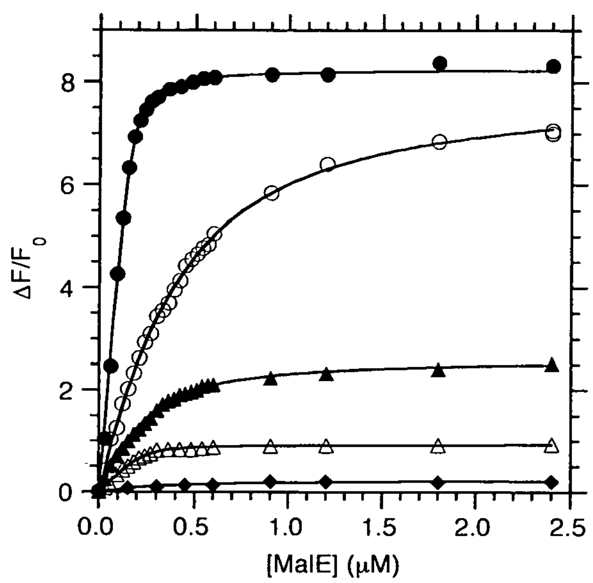


Figure 2

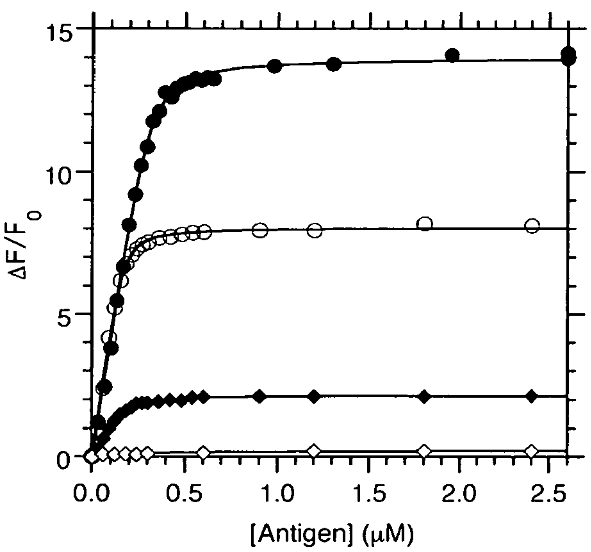


Figure 3

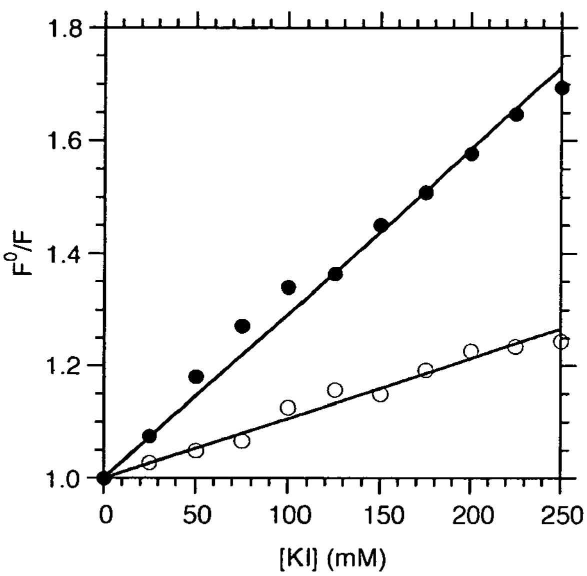


Figure 4

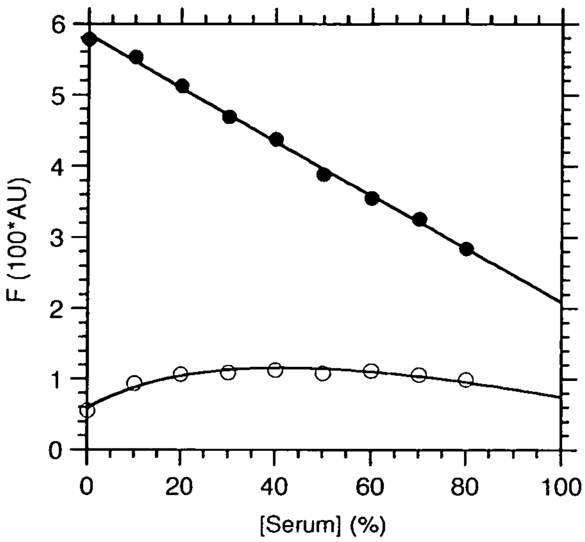


Figure 5

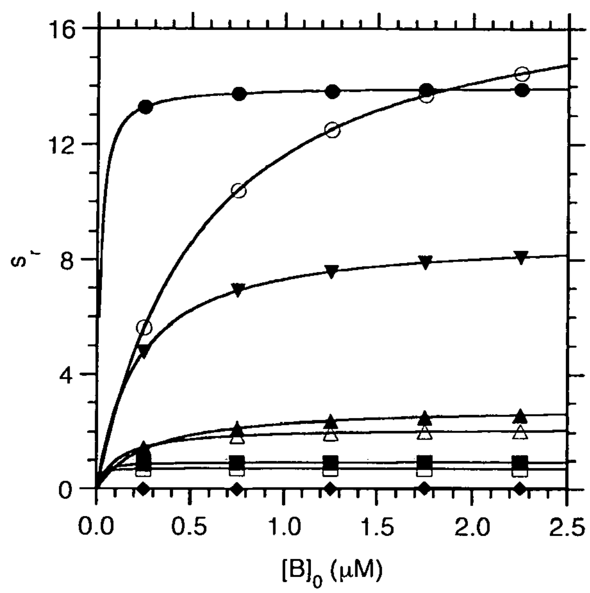


Figure 6

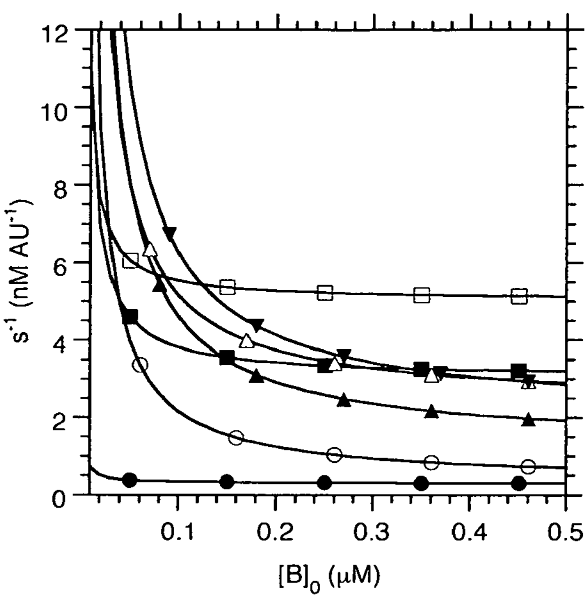


Figure 7

| | | | | | | | |
|---|---|---|---|---|---|---|---|
| No: | 2, | 3, | 5, | 13, | 14, | 26, | 33 |
| N-Cap: | | | | 23,<br>S3 | 24, | 36, | 43 |
| AR1: | 45,<br>S3 | 46,<br>S2 | 48,<br>S1 | 56,<br>S1 | 57, | 69, | 76 |
| AR2: | 78,<br>S1 | 79,<br>S1 | 81,<br>S1 | 89,<br>S1 | 90,<br>S1 | 102, | 109 |
| AR3: | 111,<br>S3 | 112,<br>S3 | 114,<br>S3 | 122,<br>S2 | 123,<br>S1 | 135, | 142 |
| C-Cap: | 144, | 145, | 147, | 155, | 156 | | |

Figure 8

European Patent Office

# EUROPEAN SEARCH REPORT

Application Number
EP 08 29 0262

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| D,Y | WO 01/65258 A (PASTEUR INSTITUT; CENTRE NAT RECH SCIENT; RENARD M) 7 September 2001 (2001-09-07) * the whole document * ----- | 1-43 | INV. G01N33/542 G01N33/533 |
| D,Y | BINZ H K ET AL: "High-affinity binders selected from designed ankyrin repeat protein libraries" NATURE BIOTECHNOL, vol. 22, no. 5, 1 May 2004 (2004-05-01), pages 575-582, XP002343919 * the whole document * ----- | 1-43 | |
| Y | WO 02/20565 A (UNIV ZUERICH; STUMPP M T; FORRER P) 14 March 2002 (2002-03-14) * the whole document * ----- | 1-43 | |
| Y | BINZ ET AL: "Engineered proteins as specific binding reagents" CURRENT OPIN BIOTECHNOL, vol. 16, no. 4, 1 August 2005 (2005-08-01), pages 459-469, XP005006173 * the whole document * ----- | 1-43 | TECHNICAL FIELDS SEARCHED (IPC) G01N |
| D,Y | BINZ H K ET AL: "Engineering novel binding proteins from nonimmunoglobulin domains" NATURE BIOTECHNOL, vol. 23, no. 10, 1 October 2005 (2005-10-01), pages 1257-1268, XP002381839 * the whole document * ----- -/-- | 1-43 | |

The present search report has been drawn up for all claims

| Place of search | Date of completion of the search | Examiner |
|---|---|---|
| Munich | 4 August 2008 | Weber, Peter |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

2

EPO FORM 1503 03.82 (P04C01)

European Patent Office

# EUROPEAN SEARCH REPORT

Application Number
EP 08 29 0262

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CHE A ET AL: "Restriction by ankyrin of band 3 rotational mobility in human erythrocyte membranes and reconstituted lipid vesicles." BIOCHEM, vol. 36, no. 31, 5 August 1997 (1997-08-05), pages 9588-9595, XP002490913 * the whole document * ----- | 1-43 | |
| | | | TECHNICAL FIELDS SEARCHED (IPC) |

The present search report has been drawn up for all claims

| Place of search | Date of completion of the search | Examiner |
|---|---|---|
| Munich | 4 August 2008 | Weber, Peter |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................................
& : member of the same patent family, corresponding document

2

EPO FORM 1503 03.82 (P04C01)

## ANNEX TO THE EUROPEAN SEARCH REPORT ON EUROPEAN PATENT APPLICATION NO. EP 08 29 0262

This annex lists the patent family members relating to the patent documents cited in the above-mentioned European search report.
The members are as contained in the European Patent Office EDP file on
The European Patent Office is in no way liable for these particulars which are merely given for the purpose of information.

04-08-2008

| Patent document cited in search report | Publication date | Patent family member(s) | Publication date |
|---|---|---|---|
| WO 0165258 A | 07-09-2001 | AT 348335 T | 15-01-2007 |
| | | AU 4074201 A | 12-09-2001 |
| | | DE 60125145 T2 | 20-09-2007 |
| | | EP 1259809 A1 | 27-11-2002 |
| | | FR 2805820 A1 | 07-09-2001 |
| | | US 2003153012 A1 | 14-08-2003 |
| WO 0220565 A | 14-03-2002 | AU 1816602 A | 22-03-2002 |
| | | CA 2421447 A1 | 14-03-2002 |
| | | JP 2004508033 T | 18-03-2004 |
| | | US 2004132028 A1 | 08-07-2004 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2001065258 A

### Non-patent literature cited in the description


- **Tatutsova ; Madden.** *FEMS Microbiol Lett.,* 1999, vol. 174, 247-250
- **Altschuh, D. ; Oncul, S. ; Demchenko, A.P.** Fluorescence sensing of intermolecular interactions and development of direct molecular biosensors. *J Mol Recognit,* 2006, vol. 19, 459-477
- **Amstutz, P. ; Binz, H.K. ; Parizek, P. ; Stumpp, M.T. ; Kohl, A. ; Grutter, M.G. ; Forrer, P. ; Pluckthun, A.** Intracellular kinase inhibitors selected from combinatorial libraries of designed ankyrin repeat proteins. *J Biol Chem,* 2005, vol. 280, 24715-24722
- **Binz, H.K. ; Amstutz, P. ; Kohl, A. ; Stumpp, M.T. ; Briand, C. ; Forrer, P. ; Grutter, M.G. ; Pluckthun, A.** High-affinity binders selected from designed ankyrin repeat protein libraries. *Nat Biotechnol,* 2004, vol. 22, 575-582
- **Binz, H.K. ; Amstutz, P. ; Pluckthun, A.** Engineering novel binding proteins from nonimmunoglobulin domains. *Nat Biotechnol,* 2005, vol. 23, 1257-1268
- **Binz, H.K. ; Stumpp, M.T. ; Forrer, P. ; Amstutz, P. ; Pluckthun, A.** Designing repeat proteins: well-expressed, soluble and stable proteins from combinatorial libraries of consensus ankyrin repeat proteins. *J Mol Biol,* 2003, vol. 332, 489-503
- **Bullock, W.O. ; Fernandez, J.M. ; Short, J.M.** XL1-Blue: a high efficiency plasmid transforming recA Escherichia coli strain with beta-galactosidase selection. *BioTechniques,* 1987, vol. 5, 376-379
- **de Lorimier, R.M. ; Smith, J.J. ; Dwyer, M.A. ; Looger, L.L. ; Sali, K.M. ; Paavola, C.D. ; Rizk, S.S. ; Sadigov, S. ; Conrad, D.W. ; Loew, L. et al.** Construction of a fluorescent biosensor family. *Protein Sci,* 2002, vol. 11, 2655-2675
- **de Lorimier, R.M. ; Tian, Y. ; Hellinga, H.W.** Binding and signaling of surface-immobilized reagentless fluorescent biosensors derived from periplasmic binding proteins. *Protein Sci,* 2006, vol. 15, 1936-1944
- **England, P. ; Bregegere, F. ; Bedouelle, H.** Energetic and kinetic contributions of contact residues of antibody D1.3 in the interaction with lysozyme. *Biochemistry,* 1997, vol. 36, 164-172
- **Foote, J. ; Winter, G.** Antibody framework residues affecting the conformation of the hypervariable loops. *J Mol Biol,* 1992, vol. 224, 487-499
- **Griffiths, D. ; Hall, G.** Biosensors--what real progress is being made?. *Trends Biotechnol,* 1993, vol. 11, 122-130
- **Harlow, E. ; Lane, D.** Antibodies: a laboratory manual. Cold Spring Harbor Laboratory, 1988, xiii, 726
- **Huber, T. ; Steiner, D. ; Rothlisberger, D. ; Pluckthun, A.** In vitro selection and characterization of DARPins and Fab fragments for the co-crystallization of membrane proteins: The Na(+)-citrate symporter CitS as an example. *J Struct Biol,* 2007, vol. 159, 206-221
- **Kawe, M. ; Forrer, P. ; Amstutz, P. ; Pluckthun, A.** Isolation of intracellular proteinase inhibitors derived from designed ankyrin repeat proteins by genetic screening. *J. Biol. Chem.,* 2006, vol. 281, 40252-40263
- **Jespers, L. ; Bonnert, T.P. ; Winter, G.** Selection of optical biosensors from chemisynthetic antibody libraries. *Protein Eng Des Sel,* 2004, vol. 17, 709-713
- **Kohl, A. ; Binz, H.K. ; Forrer, P. ; Stumpp, M.T. ; Pluckthun, A. ; Grutter, M.G.** Designed to be stable: crystal structure of a consensus ankyrin repeat protein. *Proc Natl Acad Sci U S A,* 2003, vol. 100, 1700-1705
- **Lakowicz, J.R.** Principles of fluorescent spectroscopy. Kluwer Academic/Plenum, 1999
- **Li, J. ; Mahajan, A. ; Tsai, M.D.** Ankyrin repeat: a unique motif mediating protein-protein interactions. *Biochemistry,* 2006, vol. 45, 15168-15178
- **Lisova, O. ; Hardy, F. ; Petit, V. ; Bedouelle, H.** Mapping to completeness and transplantation of a group-specific, discontinuous, neutralizing epitope in the envelope protein of dengue virus. *J Gen Virol,* 2007, vol. 88, 2387-2397
- **Lowe, C.R.** *Biosensors. Trends Biotechnol,* 1984, vol. 2, 59-65
- **Mathonet, P. ; Fastrez, J.** Engineering of non-natural receptors. *Curr Opin Struct Biol,* 2004, vol. 14, 505-511

- **Morgan, C.L. ; Newman, D.J. ; Price, C.P.** Immunosensors: technology and opportunities in laboratory medicine. *Clin Chem,* 1996, vol. 42, 193-209
- **Mosavi, L.K. ; Cammett, T.J. ; Desrosiers, D.C. ; Peng, Z.Y.** The ankyrin repeat as molecular architecture for protein recognition. *Protein Sci,* 2004, vol. 13, 1435-1448
- **Mosavi, L.K. ; Minor, D.L., Jr. ; Peng, Z.Y.** Consensus-derived structural determinants of the ankyrin repeat motif. *Proc Natl Acad Sci U S A,* 2002, vol. 99, 16029-16034
- **Nieba, L. ; Krebber, A. ; Pluckthun, A.** Competition BIAcore for measuring true affinities: large differences from values determined from binding kinetics. *Anal Biochem,* 1996, vol. 234, 155-165
- **Pace, C.N. ; Vajdos, F. ; Fee, L. ; Grimsley, G. ; Gray, T.** How to measure and predict the molar absorption coefficient of a protein. *Protein Sci,* 1995, vol. 4, 2411-2423
- **Renard, M. ; Bedouelle, H.** Improving the sensitivity and dynamic range of reagentless fluorescent immunosensors by knowledge-based design. *Biochemistry,* 2004, vol. 43, 15453-15462
- **Renard, M. ; Belkadi, L. ; Bedouelle, H.** Deriving topological constraints from functional data for the design of reagentless fluorescent immunosensors. *J Mol Biol,* 2003, vol. 326, 167-175
- **Renard, M. ; Belkadi, L. ; Hugo, N. ; England, P. ; Altschuh, D. ; Bedouelle, H.** Knowledge-based design of reagentless fluorescent biosensors from recombinant antibodies. *J Mol Biol,* 2002, vol. 318, 429-442
- **Rich, R.L. ; Myszka, D.G.** Survey of the year 2004 commercial optical biosensor literature. *J Mol Recognit,* 2005, vol. 18, 431-478
- **Rondard, P. ; Bedouelle, H.** A mutational approach shows similar mechanisms of recognition for the isolated and integrated versions of a protein epitope. *J Biol Chem,* 1998, vol. 273, 34753-34759
- **Smith, J.J. ; Conrad, D.W. ; Cuneo, M.J. ; Hellinga, H.W.** Orthogonal site-specific protein modification by engineering reversible thiol protection mechanisms. *Protein Sci,* 2005, vol. 14, 64-73
- **Smith, P.A. ; Tripp, B.C. ; DiBlasio-Smith, E.A. ; Lu, Z. ; LaVallie, E.R. ; McCoy, J.M.** A plasmid expression system for quantitative in vivo biotinylation of thioredoxin fusion proteins in Escherichia coli. *Nucleic Acids Res,* 1998, vol. 26, 1414-1420
- **Sowdhamini, R. ; Srinivasan, N. ; Shoichet, B. ; Santi, D.V. ; Ramakrishnan, C. ; Balaram, P.** Stereochemical modeling of disulfide bridges. Criteria for introduction into proteins by site-directed mutagenesis. *Protein Eng,* 1989, vol. 3, 95-103
- **Thevenot, D.R. ; Toth, K. ; Durst, R.A. ; Wilson, G.S.** Electrochemical biosensors: recommended definitions and classification. *Biosens Bioelectron,* 2001, vol. 16, 121-131
- **Vessman, J. ; Stefan, R.I. ; Van Staden, J.F. ; Danzer, K. ; Lindner, W. ; Bums, D.T. ; Fajgelj, A. ; Muller, H.** Selectivity in analytical chemistry - (IUPAC Recommendations 2001). *Pure and Applied Chemistry,* 2001, vol. 73, 1381-1386
- **Vo-Dinh, T. ; Kasili, P.** Fiber-optic nanosensors for single-cell monitoring. *Anal Bioanal Chem,* 2005, vol. 382, 918-925
- **Vriend, G.** WHAT IF: a molecular modeling and drug design program. *J Mol Graph,* 1990, vol. 8, 52-5629
- **Zahnd, C. ; Amstutz, P. ; Pluckthun, A.** Ribosome display: selecting and evolving proteins in vitro that specifically bind to a target. *Nat Methods,* 2007, vol. 4, 269-279
- **Zahnd, C. ; Pecorari, F. ; Straumann, N. ; Wyler, E. ; Pluckthun, A.** Selection and characterization of Her2 binding-designed ankyrin repeat proteins. *JBiol Chem,* 2006, vol. 281, 35167-35175
- **Zhu, H. ; Snyder, M.** Protein chip technology. *Curr Opin Chem Biol,* 2003, vol. 7, 55-63

SEQUENCE LISTING

<110> Institut Pasteur

<120> Reagentless fluorescent biosensors comprising a designed ankyrin repeat module and methods of there design and use

<130> DT - F226 - 157

<160> 10

<170> PatentIn version 3.3

<210> 1
<211> 510
<212> DNA
<213> Artificial

<220>
<223> DarpOff7 nucleotide ORF

<400> 1
```
atgagaggat cgcatcacca tcaccatcac ggatccgacc tgggtaggaa actgctggaa      60

gctgctcgtg ctggtcagga cgacgaagtt cgtatcctga tggctaacgg tgctgacgtt     120

aatgctgctg acaatactgg tactactccg ctgcacctgg ctgcttattc tggtcacctg     180

gaaatcgttg aagttctgct gaagcacggt gctgacgttg acgcttctga cgtttttggt     240

tatactccgc tgcacctggc tgcttattgg ggtcacctgg aaatcgttga agttctgctg     300

aagaacggtg ctgacgttaa cgctatggac tctgatggta tgactccact gcacctggct     360

gctaagtggg gttacctgga aatcgttgaa gttctgctga agcacggtgc tgacgttaac     420

gctcaggaca aattcggtaa gaccgctttc gacatctcca tcgacaacgg taacgaggac     480

ctggctgaaa tcctgcaaaa gcttaattag                                      510
```

<210> 2
<211> 169
<212> PRT
<213> Artificial

<220>
<223> DarpOff7 amino acid sequence

<400> 2

```
Met Arg Gly Ser His His His His His His Gly Ser Asp Leu Gly Arg
1               5                   10                  15

Lys Leu Leu Glu Ala Ala Arg Ala Gly Gln Asp Asp Glu Val Arg Ile
            20                  25                  30

Leu Met Ala Asn Gly Ala Asp Val Asn Ala Ala Asp Asn Thr Gly Thr
        35                  40                  45

Thr Pro Leu His Leu Ala Ala Tyr Ser Gly His Leu Glu Ile Val Glu
    50                  55                  60

Val Leu Leu Lys His Gly Ala Asp Val Asp Ala Ser Asp Val Phe Gly
```

65 70 75 80

Tyr Thr Pro Leu His Leu Ala Ala Tyr Trp Gly His Leu Glu Ile Val
85 90 95

Glu Val Leu Leu Lys Asn Gly Ala Asp Val Asn Ala Met Asp Ser Asp
100 105 110

Gly Met Thr Pro Leu His Leu Ala Ala Lys Trp Gly Tyr Leu Glu Ile
115 120 125

Val Glu Val Leu Leu Lys His Gly Ala Asp Val Asn Ala Gln Asp Lys
130 135 140

Phe Gly Lys Thr Ala Phe Asp Ile Ser Ile Asp Asn Gly Asn Glu Asp
145 150 155 160

Leu Ala Glu Ile Leu Gln Lys Leu Asn
165

<210> 3
<211> 33
<212> PRT
<213> Artificial

<220>
<223> Ankyrin Repeat Consensus Sequence from Mosavi et al, Protein Science, 2004

<400> 3

Asn Gly Arg Thr Pro Leu His Leu Ala Ala Arg Asn Gly His Leu Glu
1 5 10 15

Val Val Lys Leu Leu Leu Glu Ala Gly Ala Asp Val Asn Ala Lys Asp
20 25 30

Lys

<210> 4
<211> 33
<212> PRT
<213> Artificial

<220>
<223> N-terminal capping Ankyrin Repeat from Binz et al, Nat Biotechnol, 2004.

<220>
<221> misc_feature
<222> (33)..(33)
<223> Xaa can be any naturally occurring amino acid

<400> 4

Gly Ser Asp Leu Gly Lys Lys Leu Leu Glu Ala Ala Arg Ala Gly Gln

1 5 10 15

Asp Asp Glu Val Arg Ile Leu Met Ala Asn Gly Ala Asp Val Asn Ala
20 25 30

Xaa

<210> 5
<211> 25
<212> PRT
<213> Artificial

<220>
<223> C-terminal capping Ankyrin Repeat from Binz et al, Nat Biotechnol, 2004.

<400> 5

Gln Asp Lys Phe Gly Lys Thr Ala Phe Asp Ile Ser Ile Asp Asn Gly
1 5 10 15

Asn Glu Asp Leu Ala Glu Ile Leu Gln
20 25

<210> 6
<211> 4570
<212> DNA
<213> Artificial

<220>
<223> pQEMBP expression vector for MBP

<400> 6
ctcgagaaat cataaaaaat ttatttgctt tgtgagcgga taacaattat aatagattca 60

attgtgagcg gataacaatt tcacacagaa ttcattaaag aggagaaatt aactatgaga 120

ggatcgcatc accatcacca tcacggatct ggttccatga aaactgaaga aggtaaactg 180

gtaatctgga ttaacggcga taaaggctat aacggtctcg ctgaagtcgg taagaaattc 240

gagaaagata ccggaattaa agtcaccgtt gagcatccgg ataaactgga agagaaattc 300

ccacaggttg cggcaactgg cgatggccct gacattatct tctgggcaca cgaccgcttt 360

ggtggctacg ctcaatctgg cctgttggct gaaatcaccc cggacaaagc gttccaggac 420

aagctgtatc cgtttacctg ggatgccgta cgttacaacg gcaagctgat tgcttacccg 480

atcgctgttg aagcgttatc gctgatttat aacaaagatc tgctgccgaa cccgccaaaa 540

acctgggaag agatcccggc gctggataaa gaactgaaag cgaaaggtaa gagcgcgctg 600

atgttcaacc tgcaagaacc gtacttcacc tggccgctga ttgctgctga cgggggttat 660

gcgttcaagt atgaaaacgg caagtacgac attaaagacg tgggcgtgga taacgctggc 720

gcgaaagcgg gtctgacctt cctggttgac ctgattaaaa acaaacacat gaatgcagac 780

accgattact ccatcgcaga agctgccttt aataaaggcg aaacagcgat gaccatcaac 840

ggcccgtggg catggtccaa catcgacacc agcaaagtga attatggtgt aacggtactg 900

```
ccgaccttca agggtcaacc atccaaaccg ttcgttggcg tgctgagcgc aggtattaac    960
gccgccagtc cgaacaaaga gctggcaaaa gagttcctcg aaaactatct gctgactgat   1020
gaaggtctgg aagcggttaa taaagacaaa ccgctgggtg ccgtagcgct gaagtcttac   1080
gaggaagagt tggcgaaaga tccacgtatt gccgccacta tggaaaacgc ccagaaaggt   1140
gaaatcatgc cgaacatccc gcagatgtcc gctttctggt atgccgtgcg tactgcggtg   1200
atcaacgccg ccagcggtcg tcagactgtc gatgaagccc tgaaagacgc gcagactgga   1260
tccggtggta ccccgggtcg acctgcagcc aagcttaatt agctgagctt ggactcctgt   1320
tgatagatcc agtaatgacc tcagaactcc atctggattt gttcagaacg ctcggttgcc   1380
gccgggcgtt ttttattggt gagaatccaa gctagcttgg cgagattttc aggagctaag   1440
gaagctaaaa tggagaaaaa aatcactgga tataccaccg ttgatatatc ccaatggcat   1500
cgtaaagaac attttgaggc atttcagtca gttgctcaat gtacctataa ccagaccgtt   1560
cagctggata ttacggcctt tttaaagacc gtaaagaaaa ataagcacaa gttttatccg   1620
gcctttattc acattcttgc ccgcctgatg aatgctcatc cggaatttcg tatggcaatg   1680
aaagacggtg agctggtgat atgggatagt gttcaccctt gttacaccgt tttccatgag   1740
caaactgaaa cgttttcatc gctctggagt gaataccacg acgatttccg gcagtttcta   1800
cacatatatt cgcaagatgt ggcgtgttac ggtgaaaacc tggcctattt ccctaaaggg   1860
tttattgaga atatgttttt cgtctcagcc aatccctggg tgagtttcac cagttttgat   1920
ttaaacgtgg ccaatatgga caacttcttc gcccccgttt tcaccatgca tgggcaaata   1980
ttatacgcaa ggcgacaagg tgctgatgcc gctggcgatt caggttcatc atgccgtctg   2040
tgatggcttc catgtcggca gaatgcttaa tgaattacaa cagtactgcg atgagtggca   2100
gggcggggcg taattttttt aaggcagtta ttggtgccct taaacgcctg ggtaatgac    2160
tctctagctt gaggcatcaa ataaaacgaa aggctcagtc gaaagactgg gcctttcgtt   2220
ttatctgttg tttgtcggtg aacgctctcc tgagtaggac aaatccgccg ctctagagct   2280
gcctcgcgcg tttcggtgat gacggtgaaa acctctgaca catgcagctc ccggagacgg   2340
tcacagcttg tctgtaagcg gatgccggga gcagacaagc ccgtcagggc gcgtcagcgg   2400
gtgttggcgg gtgtcggggc gcagccatga cccagtcacg tagcgatagc ggagtgtata   2460
ctggcttaac tatgcggcat cagagcagat tgtactgaga gtgcaccata tgcggtgtga   2520
aataccgcac agatgcgtaa ggagaaaata ccgcatcagg cgctcttccg cttcctcgct   2580
cactgactcg ctgcgctcgg tctgtcggct gcggcgagcg gtatcagctc actcaaaggc   2640
ggtaatacgg ttatccacag aatcagggga taacgcagga aagaacatgt gagcaaaagg   2700
ccagcaaaag gccaggaacc gtaaaaaggc cgcgttgctg gcgtttttcc ataggctccg   2760
cccccctgac gagcatcaca aaaatcgacg ctcaagtcag aggtggcgaa acccgacagg   2820
actataaaga taccaggcgt ttccccctgg aagctccctc gtgcgctctc ctgttccgac   2880
cctgccgctt accggatacc tgtccgcctt tctcccttcg ggaagcgtgg cgctttctca   2940
```

atgctcacgc tgtaggtatc tcagttcggt gtaggtcgtt cgctccaagc tgggctgtgt 3000
gcacgaaccc cccgttcagc ccgaccgctg cgccttatcc ggtaactatc gtcttgagtc 3060
caacccggta agacacgact tatcgccact ggcagcagcc actggtaaca ggattagcag 3120
agcgaggtat gtaggcggtg ctacagagtt cttgaagtgg tggcctaact acggctacac 3180
tagaaggaca gtatttggta tctgcgctct gctgaagcca gttaccttcg gaaaaagagt 3240
tggtagctct tgatccggca aacaaaccac cgctggtagc ggtggttttt ttgtttgcaa 3300
gcagcagatt acgcgcagaa aaaaaggatc tcaagaagat cctttgatct tttctacggg 3360
gtctgacgct cagtggaacg aaaactcacg ttaagggatt ttggtcatga gattatcaaa 3420
aaggatcttc acctagatcc ttttaaatta aaaatgaagt tttaaatcaa tctaaagtat 3480
atatgagtaa acttggtctg acagttacca atgcttaatc agtgaggcac ctatctcagc 3540
gatctgtcta tttcgttcat ccatagctgc ctgactcccc gtcgtgtaga taactacgat 3600
acgggagggc ttaccatctg gccccagtgc tgcaatgata ccgcgagacc cacgctcacc 3660
ggctccagat ttatcagcaa taaaccagcc agccggaagg gccgagcgca gaagtggtcc 3720
tgcaacttta tccgcctcca tccagtctat taattgttgc cgggaagcta gagtaagtag 3780
ttcgccagtt aatagtttgc gcaacgttgt tgccattgct acaggcatcg tggtgtcacg 3840
ctcgtcgttt ggtatggctt cattcagctc cggttcccaa cgatcaaggc gagttacatg 3900
atcccccatg ttgtgcaaaa aagcggttag ctccttcggt cctccgatcg ttgtcagaag 3960
taagttggcc gcagtgttat cactcatggt tatggcagca ctgcataatt ctcttactgt 4020
catgccatcc gtaagatgct tttctgtgac tggtgagtac tcaaccaagt cattctgaga 4080
atagtgtatg cggcgaccga gttgctcttg cccggcgtca atacgggata ataccgcgcc 4140
acatagcaga actttaaaag tgctcatcat tggaaaacgt tcttcggggc gaaaactctc 4200
aaggatctta ccgctgttga gatccagttc gatgtaaccc actcgtgcac ccaactgatc 4260
ttcagcatct tttactttca ccagcgtttc tgggtgagca aaaacaggaa ggcaaaatgc 4320
cgcaaaaaag ggaataaggg cgacacggaa atgttgaata ctcatactct tcctttttca 4380
atattattga agcatttatc agggttattg tctcatgagc ggatacatat ttgaatgtat 4440
ttagaaaaat aaacaaatag gggttccgcg cacatttccc cgaaaagtgc cacctgacgt 4500
ctaagaaacc attattatca tgacattaac ctataaaaat aggcgtatca cgaggccctt 4560
tcgtcttcac 4570

<210> 7
<211> 5189
<212> DNA
<213> Artificial

<220>
<223> pAT224 expression vector for MBP

<400> 7
ctcgagaaat cataaaaaat ttatttgctt tgtgagcgga taacaattat aatagattca 60

attgtgagcg gataacaatt tcacacagaa ttcattaaag aggagaaatt aactatggct 120
ggtctgaacg atatcttcga agctcagaaa atcgaatggc acgaaggttc catggggaaa 180
actgaagaag gtaaactggt aatctggatt aacggcgata aaggctataa cggtctcgct 240
gaagtcggta agaaattcga gaaagatacc ggaattaaag tcaccgttga gcatccggat 300
aaactggaag agaaattccc acaggttgcg gcaactggcg atggccctga cattatcttc 360
tgggcacacg accgctttgg tggctacgct caatctggcc tgttggctga aatcaccccg 420
gacaaagcgt tccaggacaa gctgtatccg tttacctggg atgccgtacg ttacaacggc 480
aagctgattg cttacccgat cgctgttgaa gcgttatcgc tgatttataa caaagatctg 540
ctgccgaacc cgccaaaaac ctgggaagag atcccggcgc tggataaaga actgaaagcg 600
aaaggtaaga gcgcgctgat gttcaacctg caagaaccgt acttcacctg gccgctgatt 660
gctgctgacg gggttatgc gttcaagtat gaaaacggca agtacgacat taaagacgtg 720
ggcgtggata acgctggcgc gaaagcgggt ctgaccttcc tggttgacct gattaaaaac 780
aaacacatga atgcagacac cgattactcc atcgcagaag ctgcctttaa taaaggcgaa 840
acagcgatga ccatcaacgg cccgtgggca tggtccaaca tcgacaccag caaagtgaat 900
tatggtgtaa cggtactgcc gaccttcaag ggtcaaccat ccaaaccgtt cgttggcgtg 960
ctgagcgcag gtattaacgc cgccagtccg aacaaagagc tggcaaaaga gttcctcgaa 1020
aactatctgc tgactgatga aggtctggaa gcggttaata aagacaaacc gctgggtgcc 1080
gtagcgctga agtcttacga ggaagagttg gcgaaagatc cacgtattgc cgccactatg 1140
gaaaacgccc agaaaggtga aatcatgccg aacatcccgc agatgtccgc tttctggtat 1200
gccgtgcgta ctgcggtgat caacgccgcc agcggtcgtc agactgtcga tgaagccctg 1260
aaagacgcgc agactggatc cggtggtacc ccgggtcgac ctgcagccca agcttctcat 1320
caccatcacc atcactaatg agctgagctt ggactcctgt tgatagatcc agtaatgacc 1380
tcagaactcc atctggattt gttcagaacg ctcggttgcc gccgggcgtt ttttattggt 1440
gagaatccaa gctagcagta ctgcgatgag tggcagggcg gggcgtaatt tttttaaggc 1500
agttattggt gcccttaaac gcctggggta atgactctct agtttgaggc atcaaataaa 1560
acgaaaggct cagtcgaaag actgggcctt tcgttttatc tgttgtttgt cggtgaacgc 1620
tctcctgagt aggacaaatc cgccgctcta gagatttccc tcgacaattc gcgctaactt 1680
acattaattg cgttgcgctc actgcccgct ttccagtcgg gaaacctgtc gtgccagctg 1740
cattaatgaa tcggccaacg cgcggggaga ggcggtttgc gtattgggcg ccagggtggt 1800
ttttcttttc accagtgaga cgggcaacag ctgattgccc ttcaccgcct ggccctgaga 1860
gagttgcagc aagcggtcca cgctggtttg ccccagcagg cgaaaatcct gtttgatggt 1920
ggttaacggc gggatataac atgagctgtc ttcggtatcg tcgtatccca ctaccgagat 1980
atccgcacca acgcgcagcc cggactcggt aatggcgcgc attgcgccca gcgccatctg 2040
atcgttggca accagcatcg cagtgggaac gatgccctca ttcagcattt gcatggtttg 2100

ttgaaaaccg gacatggcac tccagtcgcc ttcccgttcc gctatcggct gaatttgatt 2160
gcgagtgaga tatttatgcc agccagccag acgcagacgc gccgagacag aacttaatgg 2220
gcccgctaac agcgcgattt gctggtgacc caatgcgacc agatgctcca cgcccagtcg 2280
cgtaccgtct tcatgggaga aaataatact gttgatgggt gtctggtcag agacatcaag 2340
aaataacgcc ggaacattag tgcaggcagc ttccacagca atggcatcct ggtcatccag 2400
cggatagtta atgatcagcc cactgacgcg ttgcgcgaga agattgtgca ccgccgcttt 2460
acaggcttcg acgccgcttc gttctaccat cgacaccacc acgctggcac ccagttgatc 2520
ggcgcgagat ttaatcgccg cgacaatttg cgacggcgcg tgcagggcca gactggaggt 2580
ggcaacgcca atcagcaacg actgtttgcc cgccagttgt tgtgccacgc ggttgggaat 2640
gtaattcagc tccgccatcg ccgcttccac tttttcccgc gttttcgcag aaacgtggct 2700
ggcctggttc accacgcggg aaacggtctg ataagagaca ccggcatact ctgcgacatc 2760
gtataacgtt actggtttca cattcaccac cctgaattga ctctcttccg ggcgctatca 2820
tgccataccg cgaaaggttt tgcaccttc gatggtgtca acgtaaatgc atgccgcttc 2880
gccttcccta gctagagctg cctcgcgcgt ttcggtgatg acggtgaaaa cctctgacac 2940
atgcagctcc cggagacggt cacagcttgt ctgtaagcgg atgccgggag cagacaagcc 3000
cgtcagggcg cgtcagcggg tgttggcggg tgtcggggcg cagccatgac ccagtcacgt 3060
agcgatagcg gagtgtatac tggcttaact atgcggcatc agagcagatt gtactgagag 3120
tgcaccatat gcggtgtgaa ataccgcaca gatgcgtaag gagaaaatac cgcatcaggc 3180
gctcttccgc ttcctcgctc actgactcgc tgcgctcggt ctgtcggctg cggcgagcgg 3240
tatcagctca ctcaaaggcg gtaatacggt tatccacaga atcaggggat aacgcaggaa 3300
agaacatgtg agcaaaaggc cagcaaaagg ccaggaaccg taaaaaggcc gcgttgctgg 3360
cgtttttcca taggctccgc ccccctgacg agcatcacaa aaatcgacgc tcaagtcaga 3420
ggtggcgaaa cccgacagga ctataaagat accaggcgtt tccccctgga agctccctcg 3480
tgcgctctcc tgttccgacc ctgccgctta ccggatacct gtccgccttt ctcccttcgg 3540
gaagcgtggc gctttctcaa tgctcacgct gtaggtatct cagttcggtg taggtcgttc 3600
gctccaagct gggctgtgtg cacgaacccc ccgttcagcc cgaccgctgc gccttatccg 3660
gtaactatcg tcttgagtcc aacccggtaa gacacgactt atcgccactg gcagcagcca 3720
ctggtaacag gattagcaga gcgaggtatg taggcggtgc tacagagttc ttgaagtggt 3780
ggcctaacta cggctacact agaaggacag tatttggtat ctgcgctctg ctgaagccag 3840
ttaccttcgg aaaaagagtt ggtagctctt gatccggcaa acaaaccacc gctggtagcg 3900
gtggtttttt tgtttgcaag cagcagatta cgcgcagaaa aaaaggatct caagaagatc 3960
ctttgatctt ttctacgggg tctgacgctc agtggaacga aaactcacgt taagggattt 4020
tggtcatgag attatcaaaa aggatcttca cctagatcct tttaaattaa aaatgaagtt 4080
ttaaatcaat ctaaagtata tatgagtaaa cttggtctga cagttaccaa tgcttaatca 4140

```
gtgaggcacc tatctcagcg atctgtctat ttcgttcatc catagctgcc tgactccccg    4200
tcgtgtagat aactacgata cgggagggct taccatctgg ccccagtgct gcaatgatac    4260
cgcgagaccc acgctcaccg gctccagatt tatcagcaat aaaccagcca gccggaaggg    4320
ccgagcgcag aagtggtcct gcaactttat ccgcctccat ccagtctatt aattgttgcc    4380
gggaagctag agtaagtagt tcgccagtta atagtttgcg caacgttgtt gccattgcta    4440
caggcatcgt ggtgtcacgc tcgtcgtttg gtatggcttc attcagctcc ggttcccaac    4500
gatcaaggcg agttacatga tcccccatgt tgtgcaaaaa agcggttagc tccttcggtc    4560
ctccgatcgt tgtcagaagt aagttggccg cagtgttatc actcatggtt atggcagcac    4620
tgcataattc tcttactgtc atgccatccg taagatgctt ttctgtgact ggtgagtact    4680
caaccaagtc attctgagaa tagtgtatgc ggcgaccgag ttgctcttgc ccggcgtcaa    4740
tacgggataa taccgcgcca catagcagaa ctttaaaagt gctcatcatt ggaaaacgtt    4800
cttcggggcg aaaactctca aggatcttac cgctgttgag atccagttcg atgtaaccca    4860
ctcgtgcacc caactgatct tcagcatctt ttactttcac cagcgtttct gggtgagcaa    4920
aaacaggaag gcaaaatgcc gcaaaaaagg gaataagggc gacacggaaa tgttgaatac    4980
tcatactctt cctttttcaa tattattgaa gcatttatca gggttattgt ctcatgagcg    5040
gatacatatt tgaatgtatt tagaaaaata aacaaatagg ggttccgcgc acatttcccc    5100
gaaaagtgcc acctgacgtc taagaaacca ttattatcat gacattaacc tataaaaata    5160
ggcgtatcac gaggcccttt cgtcttcac                                      5189
```

<210> 8
<211> 33
<212> PRT
<213> Artificial

<220>
<223> Designed AR module from Binz et al, Nat Biotechnol, 2004.

<220>
<221> misc_feature
<222> (2)..(3)
<223> Xaa can be any naturally occurring amino acid

<220>
<221> misc_feature
<222> (5)..(5)
<223> Xaa can be any naturally occurring amino acid

<220>
<221> misc_feature
<222> (13)..(14)
<223> Xaa can be any naturally occurring amino acid

<220>
<221> misc_feature
<222> (26)..(26)
<223> Xaa can be any naturally occurring amino acid

<220>

<221> misc_feature
<222> (33)..(33)
<223> Xaa can be any naturally occurring amino acid

<400> 8

Asp Xaa Xaa Gly Xaa Thr Pro Leu His Leu Ala Ala Xaa Xaa Gly His
1 5 10 15

Leu Glu Ile Val Glu Val Leu Leu Lys Xaa Gly Ala Asp Val Asn Ala
20 25 30

Xaa

<210> 9
<211> 3887
<212> DNA
<213> Artificial

<220>
<223> pQEOff7 plasmid

<400> 9
ctcgagaaat cataaaaaat ttatttgctt tgtgagcgga taacaattat aatagattca 60
attgtgagcg gataacaatt tcacacagaa ttcattaaag aggagaaatt aactatgaga 120
ggatcgcatc accatcacca tcacggatcc gacctgggta ggaaactgct ggaagctgct 180
cgtgctggtc aggacgacga agttcgtatc ctgatggcta acggtgctga cgttaatgct 240
gctgacaata ctggtactac tccgctgcac ctggctgctt attctggtca cctggaaatc 300
gttgaagttc tgctgaagca cggtgctgac gttgacgctt ctgacgtttt tggttatact 360
ccgctgcacc tggctgctta ttggggtcac ctggaaatcg ttgaagttct gctgaagaac 420
ggtgctgacg ttaacgctat ggactctgat ggtatgactc cactgcacct ggctgctaag 480
tggggttacc tggaaatcgt tgaagttctg ctgaagcacg gtgctgacgt taacgctcag 540
gacaaattcg gtaagaccgc tttcgacatc tccatcgaca acggtaacga ggacctggct 600
gaaatcctgc aaaagcttaa ttagctgagc ttggactcct gttgatagat ccagtaatga 660
cctcagaact ccatctggat ttgttcagaa cgctcggttg ccgccgggcg tttttttattg 720
gtgagaatcc aagctagctt ggcgagattt tcaggagcta aggaagctaa aatggagaaa 780
aaaatcactg gatataccac cgttgatata tcccaatggc atcgtaaaga acattttgag 840
gcatttcagt cagttgctca atgtacctat aaccagaccg ttcagctgga tattacggcc 900
tttttaaaga ccgtaaagaa aaataagcac aagttttatc cggcctttat tcacattctt 960
gcccgcctga tgaatgctca tccggaattt cgtatggcaa tgaaagacgg tgagctggtg 1020
atatgggata gtgttcaccc ttgttacacc gttttccatg agcaaactga aacgttttca 1080
tcgctctgga gtgaatacca cgacgatttc cggcagtttc tacacatata ttcgcaagat 1140
gtggcgtgtt acggtgaaaa cctggcctat ttccctaaag ggtttattga gaatatgttt 1200
ttcgtctcag ccaatccctg ggtgagtttc accagttttg atttaaacgt ggccaatatg 1260

gacaacttct tcgcccccgt tttcaccatg ggcaaatatt atacgcaagg cgacaaggtg 1320
ctgatgccgc tggcgattca ggttcatcat gccgtttgtg atggcttcca tgtcggcaga 1380
atgcttaatg aattacaaca gtactgcgat gagtggcagg gcggggcgta attttttaa 1440
ggcagttatt ggtgccctta aacgcctggg gtaatgactc tctagcttga ggcatcaaat 1500
aaaacgaaag gctcagtcga aagactgggc ctttcgtttt atctgttgtt tgtcggtgaa 1560
cgctctcctg agtaggacaa atccgccctc tagagctgcc tcgcgcgttt cggtgatgac 1620
ggtgaaaacc tctgacacat gcagctcccg gagacggtca cagcttgtct gtaagcggat 1680
gccgggagca gacaagcccg tcagggcgcg tcagcgggtg ttggcgggtg tcggggcgca 1740
gccatgaccc agtcacgtag cgatagcgga gtgtatactg gcttaactat gcggcatcag 1800
agcagattgt actgagagtg caccatatgc ggtgtgaaat accgcacaga tgcgtaagga 1860
gaaaataccg catcaggcgc tcttccgctt cctcgctcac tgactcgctg cgctcggtcg 1920
ttcggctgcg gcgagcggta tcagctcact caaaggcggt aatacggtta tccacagaat 1980
caggggataa cgcaggaaag aacatgtgag caaaaggcca gcaaaaggcc aggaaccgta 2040
aaaaggccgc gttgctggcg tttttccata ggctccgccc ccctgacgag catcacaaaa 2100
atcgacgctc aagtcagagg tggcgaaacc cgacaggact ataaagatac caggcgtttc 2160
cccctggaag ctccctcgtg cgctctcctg ttccgaccct gccgcttacc ggatacctgt 2220
ccgcctttct cccttcggga agcgtggcgc tttctcatag ctcacgctgt aggtatctca 2280
gttcggtgta ggtcgttcgc tccaagctgg gctgtgtgca cgaacccccc gttcagcccg 2340
accgctgcgc cttatccggt aactatcgtc ttgagtccaa cccggtaaga cacgacttat 2400
cgccactggc agcagccact ggtaacagga ttagcagagc gaggtatgta ggcggtgcta 2460
cagagttctt gaagtggtgg cctaactacg gctacactag aaggacagta tttggtatct 2520
gcgctctgct gaagccagtt accttcggaa aaagagttgg tagctcttga tccggcaaac 2580
aaaccaccgc tggtagcggt ggtttttttg tttgcaagca gcagattacg cgcagaaaaa 2640
aaggatctca agaagatcct ttgatctttt ctacggggtc tgacgctcag tggaacgaaa 2700
actcacgtta agggattttg gtcatgagat tatcaaaaag gatcttcacc tagatccttt 2760
taaattaaaa atgaagtttt aaatcaatct aaagtatata tgagtaaact tggtctgaca 2820
gttaccaatg cttaatcagt gaggcaccta tctcagcgat ctgtctattt cgttcatcca 2880
tagttgcctg actccccgtc gtgtagataa ctacgatacg ggagggctta ccatctggcc 2940
ccagtgctgc aatgataccg cgagacccac gctcaccggc tccagattta tcagcaataa 3000
accagccagc cggaagggcc gagcgcagaa gtggtcctgc aactttatcc gcctccatcc 3060
agtctattaa ttgttgccgg gaagctagag taagtagttc gccagttaat agtttgcgca 3120
acgttgttgc cattgctaca ggcatcgtgg tgtcacgctc gtcgtttggt atggcttcat 3180
tcagctccgg ttcccaacga tcaaggcgag ttacatgatc ccccatgttg tgcaaaaaag 3240
cggttagctc cttcggtcct ccgatcgttg tcagaagtaa gttggccgca gtgttatcac 3300

```
tcatggttat ggcagcactg cataattctc ttactgtcat gccatccgta agatgctttt   3360
ctgtgactgg tgagtactca accaagtcat tctgagaata gtgtatgcgg cgaccgagtt   3420
gctcttgccc ggcgtcaata cgggataata ccgcgccaca tagcagaact ttaaaagtgc   3480
tcatcattgg aaaacgttct tcggggcgaa aactctcaag gatcttaccg ctgttgagat   3540
ccagttcgat gtaacccact cgtgcaccca actgatcttc agcatctttt actttcacca   3600
gcgtttctgg gtgagcaaaa acaggaaggc aaaatgccgc aaaaaaggga ataagggcga   3660
cacggaaatg ttgaatactc atactcttcc tttttcaata ttattgaagc atttatcagg   3720
gttattgtct catgagcgga tacatatttg aatgtattta gaaaaataaa caaatagggg   3780
ttccgcgcac atttccccga aaagtgccac ctgacgtcta agaaaccatt attatcatga   3840
cattaaccta taaaaatagg cgtatcacga ggccctttcg tcttcac                 3887
```

```
<210>  10
<211>  510
<212>  DNA
<213>  Artificial

<220>
<223>  optimized gene coding for DarpOff7

<400>  10
atgagaggat cgcatcacca tcaccatcac ggatccgatc tcgggcgcaa actgctggag     60
gcagcccgag caggccaaga cgacgaagtg cgtattctga tggccaacgg tgctgatgtc    120
aacgctgcag ataataccgg cacgacacca ctccatttgg ccgcgtatag tggccatttg    180
gaaattgtcg aagtactctt aaaacacggg gcagatgtgg atgcatcaga cgtgttcggc    240
tacaccccgc tgcatctggc ggcatactgg ggtcacctgg agattgttga ggttttgctg    300
aagaatggcg ctgatgttaa cgcgatggat agcgatggga tgacgccttt gcacctcgca    360
gccaagtggg ggtacctcga aatcgtagaa gtcctgctta agcatggtgc ggacgtgaat    420
gctcaggaca aatttggcaa aacagcgttc gatatcagca tcgataacgg caacgaggat    480
ctggcagaaa ttttacagaa gcttaattag                                     510
```